(19) Europäisches Patentamt
European Patent Office
Office européen des brevets

(11) Veröffentlichungsnummer : **0 441 207 B1**

(12) **EUROPÄISCHE PATENTSCHRIFT**

(45) Veröffentlichungstag der Patentschrift : **30.03.94 Patentblatt 94/13**

(21) Anmeldenummer : **91101013.0**

(22) Anmeldetag : **26.01.91**

(51) Int. Cl.[5] : **C07D 213/30,** C07D 213/50, A01N 43/40, C07D 401/06, C07D 407/06, C07D 409/06

# (54) Heteroarylalkene, Verfahren und Zwischenprodukte zu ihrer Herstellung und ihre Verwendung.

Die Akte enthält technische Angaben, die nach dem Eingang der Anmeldung eingereicht wurden und die nicht in dieser Patentschrift enthalten sind.

(30) Priorität : **09.02.90 DE 4003919**

(43) Veröffentlichungstag der Anmeldung : **14.08.91 Patentblatt 91/33**

(45) Bekanntmachung des Hinweises auf die Patenterteilung : **30.03.94 Patentblatt 94/13**

(84) Benannte Vertragsstaaten : **AT BE CH DE DK ES FR GB GR IT LI NL SE**

(56) Entgegenhaltungen :
**EP-A- 0 104 690**
**EP-A- 0 214 566**
**CHEMICAL ABSTRACTS, Bnd. 108, Nr. 13, 1988, Columbus, Ohio, US; Zusammenfassung 112429B, R. NASU: 'Preparation of pyridine derivatives as pesticides'**

(56) Entgegenhaltungen :
**JOURNAL OF MEDICINAL CHEMISTRY. Bnd. 20, Nr. 11, 1977, WASHINGTON US Seiten 1400 - 1408; O.W. WOLTERSDORF: 'Acylaryloxy acetic acid diuretics' Seite 1404, "compound" 25e**
**Chemical Abstracts 78, 147754t. (1973)**

(73) Patentinhaber : **BASF Aktiengesellschaft**
**Carl-Bosch-Strasse 38**
**D-67063 Ludwigshafen (DE)**

(72) Erfinder : **Zipperer, Bernhard, Dr.**
**Am Hergottsacker 6**
**W-6716 Dirmstein (DE)**
Erfinder : **Lauer, Manfred, Dr.**
**Im Zinkig 114**
**W-6700 Ludwigshafen (DE)**
Erfinder : **Goetz, Norbert, Dr.**
**Schoefferstrasse 25**
**W-6520 Worms 1 (DE)**
Erfinder : **Zierke, Thomas Dr.**
**Akazienstrasse 12**
**W-6737 Boehl-Iggelheim (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Heteroarylalkene, Verfahren und Zwischenprodukte zu ihrer Herstellung und ihre Verwendung

Die vorliegende Erfindung betrifft neue Heteroarylalkene der allgemeinen Formel I,

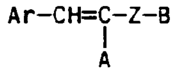

I

in der die Substituenten folgende Bedeutung haben:

Ar 3-Pyridyl;

A $C_1$-$C_6$-Alkyl, $C_2$-$C_6$-Alkenyl, $C_3$-$C_8$-Cycloalkyl, ($C_3$-$C_8$)-Cycloalkyl-($C_1$-$C_3$)-alkyl, ein-, zwei- oder dreikerniges Aryl oder Aryl-$C_1$-$C_3$-alkyl, wobei Aryl jeweils ein-, zwei- oder dreifach durch die Reste $C_1$-$C_4$-Alkyl, $C_1$-$C_4$-Alkoxy, $C_1$-$C_4$-Mono-, Di- oder Trihalogenalkyl, $C_1$-$C_4$-Halogen-alkoxy, Phenyl oder Halogen substituiert sein kann;

Z

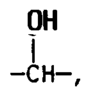

B ein- oder zweikerniges Aryl oder Benzyl bedeutet,

in denen die aromatischen Ringe jeweils ein-, zwei- oder dreifach durch $C_1$-$C_4$-Alkyl, $C_1$-$C_4$-Alkoxy, $C_1$-$C_4$-Halogenalkyl, $C_1$-$C_4$-Halogenalkoxy, Phenyl, Halogenphenyl oder Halogen substituiert sein können;

sowie deren N-Oxide und Säureadditionssalze mit anorganischen Mineralsäuren, Carbonsäuren oder einkernigen Arylsulfonsäuren.

Des weiteren betrifft die Erfindung Verfahren zur Herstellung dieser Verbindungen, weiterhin Zwischenprodukte, Verfahren zur Herstellung der Zwischenprodukte, die Heteroarylalkene I enthaltende fungizide Mittel, sowie ein Verfahren zur Bekämpfung von Schadpilzen mit Hilfe der Heteroarylalkene I bzw. dieser fungiziden Mittel.

Fungizide Heteroarylalkene, z.B.

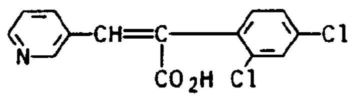

,

sind aus der EP-A-2 104 690 bekannt. Es ist ferner bekannt, ein 1,3-Dipyridyl-propenon (C.A. 108, 112 429b (1988)) oder 3-Propenylpyridine (EP 214 566) als Fungizide zu verwenden. Allerdings ist die fungizide Wirkung der meisten dieser Verbindungen unbefriedigend. Es sind ferner die Verbindungen Pyridylbutenylketone (J. Med. Chem. 20, 1400-1408 (1977)) und Dipyridylethenylketone (C.A. 78, 147 754 t (1973)) bekannt.

Der Erfindung lag daher die Aufgabe zugrunde, besonders aktive fungizide Verbindungen bereitzustellen.

Demgemäß wurden die eingangs definierten Heteroarylalkene I gefunden.

Im Hinblick auf die bestimmungsgemäße Verwendung der Heteroarylalkene I als Fungizide kommen als Substituenten vorzugsweise folgende Reste in Betracht:

Ar 3-Pyridyl,

A Methyl, Ethyl, n-Propyl, Isopropyl, n-Butyl, Isobutyl, tert.-Butyl, n-Pentyl, Isopentyl, neo-Pentyl, n-Hexyl, neo-Hexyl; Cyclopropyl, Cyclopentyl, Cyclohexyl, Cycloheptyl, Cyclooctyl, Cyclopropylmethyl, Cyclopentylmethyl, Cyclohexylmethyl, 2-Cyclohexylethyl, 3-Cyclohexylpropyl; Phenyl, Mono-, Di- oder Trimethylphenyl, tert.-Butylphenyl, Mono-, Di- oder Trimethoxyphenyl, n-, tert.-Butoxyphenyl, Trifluormethylphenyl, Difluormethoxyphenyl, Trifluormethoxyphenyl, Tetrafluorethoxyphenyl, Biphenyl, Mono-, Di- oder Trifluorphenyl, Mono-, Di-, Trichlorphenyl, Chlorfluorphenyl, Benzyl, Mono-, Di-, Trimethylbenzyl, Mono-, Di-, Trichlorbenzyl, Fluorbenzyl, 2-Phenylethyl, Mono-, Di-, Trimethylphenylethyl, Mono-, Di-, Trichlorphenylethyl, Fluorphenylethyl, 3-Phenylpropyl

B Phenyl, Mono-, Di- oder Trimethylphenyl, Ethylphenyl, Isopropylphenyl, tert.-Butylphenyl, Naphthyl, Mono-, Di- oder Trimethoxyphenyl, n- oder tert.-Butoxyphenyl, Trifluormethylphenyl,

Difluormethoxyphenyl, Trifluormethoxyphenyl, Tetrafluorethoxyphenyl, Biphenyl, Chlorbiphenyl, Mono-, Di- oder Trichlorphenyl, Mono-, Di- oder Trifluorphenyl, Chlorfluorphenyl, Bromphenyl, Benzyl, Mono-, Di- oder Trimethylbenzyl, Fluorbenzyl, Mono-, Di- oder Trichlorbenzyl, Chlorfluorbenzyl,

Z

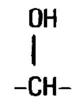 .

Besonders bevorzugt sind Verbindungen mit den Resten A Ethyl, n-Propyl, n-Butyl, tert.-Butyl, n-Pentyl, neo-Pentyl, n-Hexyl, Phenyl, 4-Fluorphenyl;

B Phenyl, 2-Chlorphenyl, 4-Chlorphenyl, 2,4-Dichlorphenyl, 4-Fluorphenyl, 2-Methylphenyl, 4-Methylphenyl, 2,4-Dimethylphenyl, 2,6-Dimethylphenyl, 2,4,6-Trimethylphenyl, 4-Biphenyl; Ar 3-Pyridyl. Besonders gut geeignete Verbindungen als Fungizide sind E-1-(2,4-Dichlorphenyl)-2-(3-pyridylmethyliden)-4,4-dimethylpentan-1-ol, E-1-(2,4-Dimethylphenyl)-2-(3-pyridylmethyliden)-hexan-1-ol, E-1-(2,4-Dichlorphenyl)-2-(3-pyridylmethyliden)-3,3-dimethyl-butan-1-ol, E-1-(2,4-Dichlorphenyl)-2-(3-pyridylmethyliden)-hexan-1-ol und E-1-(2,4-Dichlorphenyl)-2-(3-pyridylmethyliden)-octan-1-ol.

Die Heteroarylalkene I enthalten eine unsymmetrisch substituierte C=C-Doppelbindung. Sie können daher als E/Z-Isomerengemische oder in Form der reinen Isomeren vorliegen. Die reinen Isomeren können durch bekannte Trennverfahren wie fraktionierte Kristallisation oder Chromatographie aus den Isomerengemischen gewonnen werden. Sowohl die einzelnen isomeren Verbindungen als auch ihre Gemische werden von der Erfindung umfaßt.

Die Heteroarylalkenole Ia erhält man beispielsweise dadurch, daß man ein Heteroarylalkenal II mit einem Lithiumorganyl Li-B oder einem Grignard-Reagenz B-MgHal, wobei Hal für Chlor, Brom, Jod steht, in an sich bekannter Weise umsetzt. Dabei erfolgt die Umsetzung in einem inerten Lösungsmittel, vorzugsweise einem Ether wie Diethylether, Tetrahydrofuran, Methyl-tert.-butylether oder einem Gemisch dieser Ether. Es empfiehlt sich hierbei, die metallorganische Verbindung vorzulegen und das Heteroarylalkenal II in flüssiger Phase bei (-70) bis 50°C zuzudosieren. Bei den Li-Organylen ist es vorteilhaft bei (-70) bis 0°C, vorzugsweise bei (-70) bis (-30°C) zu arbeiten. Die Temperaturen bei den Umsetzungen mit den Grignard-Reagenzien wählt man in der Regel zwischen 0 und 50°C, insbesondere zwischen 20 und 50°C. Man setzt die genannten Ausgangsstoffe zweckmäßig im Molverhältnis 0,5 : 1 bis 2 : 1 ein.

Die Heteroarylalkenale II, in denen A Methyl, unsubstituiertes oder substituiertes Phenyl bedeutet, sind aus J. Org. Chem. 43 (1978) 3396 und EP-A2-298 380 bekannt. Die neuen Heteroarylalkenale III, in denen Ar für 3-Pyridyl steht und D $C_2$-$C_6$-Alkyl, bevorzugt Ethyl, n-, i-Propyl, n- oder tert.-Butyl, $C_3$-$C_8$-Cycloalkyl, insbesondere Cyclopropyl, Cyclopentyl oder Cyclohexyl, ($C_3$-$C_8$)-Cycloalkyl-($C_1$-$C_3$)-alkyl, vor allem Cyclopropylmethyl, Cyclopentylmethyl, Cyclohexylmethyl, 2-Cyclohexylethyl, 3-Cyclohexylpropyl oder ein- oder zweikerniges Aryl-($C_1$-$C_3$)alkyl, bevorzugt Benzyl, Chlorbenzyl, Fluorbenzyl, 2-Phenylethyl, Chlorphenylethyl, Fluorphenylethyl oder 3-Phenylpropyl bedeutet, können analog zu dem aus EP-A2-298 380 bekannten Verfahren durch Aldolkondensation von D-$CH_2$CHO mit ArCHO hergestellt werden (s. auch Houben-Weyl, Methoden der organischen Chemie, Bd. III/1, S. 76 ff).

Die Ausgangsstoffe des Typs D-$CH_2$CHO sind für D = Alkyl leicht zugänglich, für die anderen Reste können sie nach bekannten Methoden (s. Houben-Weyl, Band 7/1, S. 55 f., 76 f., 159 f.) hergestellt werden.

3-Formyl-pyridin ist allgemein bekannt und leicht zugänglich.

Die metallorganischen Verbindungen Li-B und HalMgB lassen sich nach bekannten Verfahren wie sie in Houben-Weyl, Methoden der organischen Chemie, Bd. XIII/1, S. 87 ff und Houben-Weyl, Methoden der organischen Chemie, Bd. XIII/2a, S. 54 ff beschrieben sind aus B-Hal und Li bzw. Mg herstellen.

Eine zweite Methode, die Heteroarylalkenole Ia darzustellen, besteht darin, daß man einen Heteroarylaldehyd ArCHO mit einem Keton vom Typ

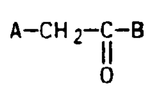

zum Heteroarylalkenon Ib umsetzt. Das Heteroarylalkenon Ib läßt sich dann mit an sich bekannten Methoden zum Heteroarylalkenol Ia reduzieren. Ar, A und B haben die in Anspruch 1 genannten Bedeutungen.

Die Aldolkondensation des Heteroarylaldehyds ArCHO mit dem Keton

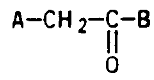

erfolgt in Gegenwart von basischen oder sauren Katalysatoren (s. Houben-Weyl, Methoden der organischen Chemie, Bd. VII/1, S. 76 ff). Als basische Katalysatoren eignen sich beispielsweise Alkali- oder Erdalkalihydroxide wie Natrium-, Kalium-, Calcium- oder Bariumhydroxid, Alkalialkoholate wie Natrium- oder Kaliummethylat, -ethylat, -propylat oder -butylat oder Amine wie Diethylamin, Diisopropylamin, Dicyclohexylamin, Pyrrolidin oder Piperidin. Als saure Katalysatoren kommen beispielsweise schwache Säuren wie Essigsäure, Oxalsäure, Borsäure, Sulfonsäuren wie Methansulfonsäure, Benzolsulfonsäure, p-Toluolsulfonsäure sowie Salze schwacher Säuren mit Aminen wie Piperidinacetat, Pyrrolidinacetat, Ethylendiamintetraacetat, Pyridinmethansulfonat oder Pyridin-p-toluol-sulfonat in Betracht.

Geeignete Lösungsmittel sind beispielsweise Alkohole wie Methanol, Ethanol, Propanol, Isopropanol, Butanol oder tert.Butanol, Ether wie Diethylether, Methyl-tert.butylether, Tetrahydrofuran oder Dioxan, oder Kohlenwasserstoffe wie Cyclohexan, Benzol, Toluol oder Xylol.

Die Reaktion führt man in der Regel bei Temperaturen von 0 bis 60°C durch.

Das Molverhältnis der Ausgangsstoffe beträgt 0,5 : 1 bis 5 : 1 (ArCHO/Keton), das Molverhältnis ArCHO zum Katalysator beträgt 1 : 1 bis 100 : 1.

Die Ausgangsverbindungen des Typs

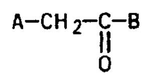

lassen sich z.B. in bekannter Weise aus Carbonsäurechloriden und Aromaten durch Friedel-Crafts-Acylierung (s.Houben-Weyl, Band 7/2a, S. 15 ff) herstellen.

Die Heteroarylalkenone Ib lassen sich mit an sich bekannten Methoden zu den bereits beschriebenen Heteroarylalkenolen Ia reduzieren (s. Houben-Weyl, Methoden der organischen Chemie, Bd. IV, 1d, S. 297 ff). Geeignete Reduktionsmittel sind beispielsweise komplexe Hydride wie Lithiumaluminiumhydrid, Lithiumtrimethoxyaluminiumhydrid, Natriumborhydrid oder Natriumcyanborhydrid sowie Aluminiumalkoholate wie Aluminiumisopropylat oder Aluminiumcyclohexanolat (s. M. Hudlicky, Reductions in Organic Chemistry, S. 119 f, Ellis Horward Series, Chichester, 1984). Als Lösungsmittel für die Reduktionen werden je nach Art des Reduktionsmittels beispielsweise Ether, wie Diethylether, Tetrahydrofuran, Dimethoxyethan oder Dioxan oder Alkohole wie Methanol, Ethanol, n-Propanol, Isopropanol oder Cyclohexanol verwendet.

Bei der Reduktion mit komplexen Hydriden empfiehlt es sich, die Reaktion bei (-30) bis 20°C durchzuführen, während bei Verwendung von Aluminiumalkoholaten höhere Temperaturen, insbesondere die Siedetemperatur des dem Aluminiumalkoholat zugrunde liegenden freien Alkohols, vorteilhaft sind. Bevorzugt ist die Verwendung von Natriumborhydrid oder Natriumcyanborhydrid in Methanol oder Ethanol. Das Äquivalentverhältnis von Heteroarylalkenon Ib zu Reduktionsmittel beträgt in der Regel 1:1 bis 1:2.

Die Oxidation der Heteroarylalkene I zu den entsprechenden N-Oxiden erfolgt nach üblichen Methoden. Als Oxidationsmittel eignen sich beispielsweise Persäuren oder in situ aus Wasserstoffperoxid und Carbonsäureanhydriden erzeugte Persäuren, Wasserstoffperoxid oder organische Derivate des Wasserstoffperoxids (s. z.B. R. A. Abramovitch und E. M. Smith in Chemistry of Heterocyclic Compounds, Band 14, Suppl. 2, Seite 1 ff; John Wiley, New York 1974; A. R. Katritzky, J. M. Lagouski, The Chemistry of Heterocyclic N-Oxides, S. 21 ff, Academic Press, New York 1971).

Die Herstellung der Säureadditionssalze von den Heteroarylalkenen I erfolgt ebenfalls nach üblichen Methoden.

Eine Übersicht über die genannten Herstellungsverfahren der Heteroarylalkene I ist in folgendem Schema gezeigt:

Schema: Übersicht über die Herstellungsverfahren der Heteroarylalkene I

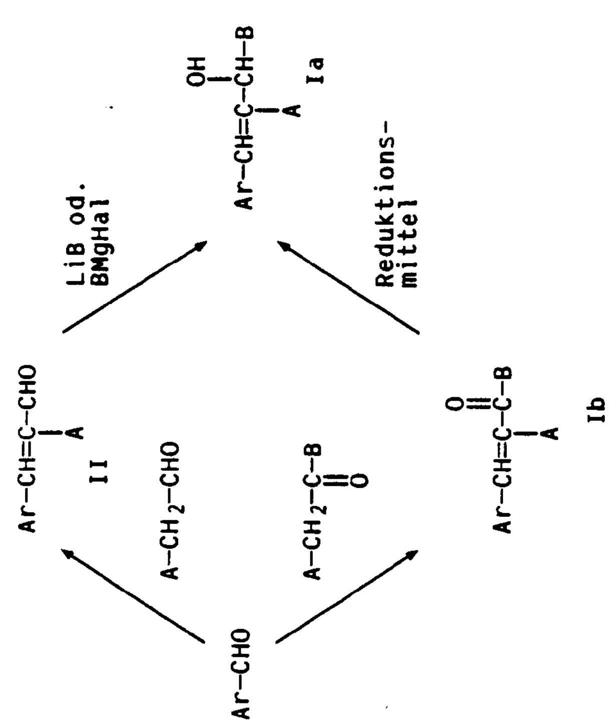


Die folgenden Beispiele erläutern die Herstellung der neuen Verbindungen:

1. Herstellung von E-1-(4-Fluorphenyl)-2-(3-pyridylmethyliden)-1-hexanol über E-2-(3-Pyridylmethyliden)-hexanal

Zu einer Lösung von 160,5 g (1,5 mol) Pyridin-3-aldehyd in 750 ml Methanol wurden 6,0 g (0,15 mol) Natriumhydroxid gegeben. Anschließend wurden bei Raumtemperatur innerhalb von 3 h 150,0 g (1,5 mol) Hexanal zugetropft. Es wurde 1 h nachgerührt, mit Essigsäure ein pH von 6 eingestellt und dann das Lösungsmittel i. Vak. verdampft. Der Rückstand wurde in einem Wasser/Dichlormethan-Gemisch aufgenommen und die organische Phase mit Wasser gewaschen, über Natriumsulfat getrocknet und i. Vak. eingeengt. Nach Destillation wurden 190 g (67 % d. Th) E-2-(3-Pyridylmethyliden)hexanal als gelbes Öl vom Sdp. 132 bis 134°C/2 mbar erhalten.

Aus 4,1 g (0,17 mol) Magnesiumspänen, die mit 1 ml 1,2-Dibrommethan aktiviert wurden, wurde durch Zutropfen einer Lösung von 29,8 g (0,17 mol) 4-Fluorbrombenzol in 200 ml Tetrahydrofuran eine Grignardlösung bereitet. Es wurde 30 min bei 50°C nachgerührt und dann 20,8 g (0,11 mol) des oben hergestellten E-2-(3-Pyridylmethyliden)hexanals in 100 ml Tetrahydrofuran zugetropft. Nach zweistündigem Rühren bei Rückflußtemperatur wurde abgekühlt, mit Wasser hydrolysiert und mit gesätt. $NH_4Cl$-Lösung auf pH 8 eingestellt. Die wäßrige Phase wurde mit Ether extrahiert, die vereinigten organischen Phasen mit Wasser gewaschen, über $Na_2SO_4$ getrocknet und i. Vak. eingedampft. Es verblieben 27,1 g E-1-(4-Fluorphenyl)-2-(3-pyridylmethyliden)-1-hexanol als gelbes Öl, Reinheit nach GC 94 %. (Ausbeute: 82 % d. Th.) (s. Tabelle, Bsp. 290).

$^1$H-NMR ($CDCl_3$) $\delta$ = 8.40, 8.30, 7.58, 7.22 (2-,6-,4-,5-H des Pyridinteils), 7.38, 7.02 (2 $H_m$ und 2 $H_o$ des

Arylteils), 6.65 (C=C<u>H</u>), 5.30 (C<u>H</u>OH), 4.5 (O<u>H</u>), 2.20, 1.95 (3-$CH_2$), 1.23 (4- und 5-$CH_2$), 0.78 (6-$CH_3$). IR (Film): $\tilde{\upsilon}$= 3200, 2957, 2931, 2870, 1603, 1507, 1222, 1155, 1028, 838, 713 $cm^{-1}$.

2. Herstellung von E-1-(2-Chlorphenyl)-2-(4-fluorphenyl)-3-(3-pyridyl)-2-propen-1-on

Eine Mischung von 24,9 g (0,10 mol) (4-Fluorbenzyl)-(2-chlorphenyl)-keton, 10,7 g (0,10 mol) Pyridin-3-aldehyd und 2,9 g (0,02 mol) Piperidinacetat in 250 ml Toluol wurde solange am Wasserabscheider erhitzt, bis sich kein Wasser mehr abtrennte. Nach dem Abkühlen wurde zweimal mit gesätt. $NaHCO_3$-Lösung und mit Wasser gewaschen, über Natriumsulfat getrocknet und i. Vak. eingeengt. Der Rückstand wurde aus Methyl-tert.butylether/Hexan (2:1) kristallisiert. Dabei wurden 16,6 g (40 % d. Th.) Produkt als gelbe Kristalle vom Schmp. 102°C erhalten.

3. Herstellung von E-1-(2-Chlorphenyl)-2-(4-fluorphenyl)-3-(3-pyridyl)-2-propen-1-ol

Zu einer Lösung von 33,75 g (0,10 mol) des unter 2.) hergestellten E-1-(2-Chlorphenyl)-2-(4-fluorphenyl)-3-(3-pyridyl)-2-propen-1-ons in 500 ml Methanol wurden bei 0°C portionsweise 7,6 g (0,20 mol) Natriumborhydrid gegeben. Man rührte 2 h bei 0°C nach, zerstörte überschüssiges Natriumborhydrid durch Zutropfen von Essigsäure und engte i. Vak. ein. Der Rückstand wurde in einem Wasser/Dichlormethan-Gemisch aufgenommen, die mit Wasser gewaschene organische Phase über Natriumsulfat getrocknet und i. Vak. eingeengt. Durch Verrühren mit Methyl-tert.butylether/Pentan (9:1) wurden 27,0 g (79 % d. Th.) kristallines Produkt vom Schmp. 82-83°C erhalten (vgl. Tabelle, Bsp. Nr. 682).

$^1H$-NMR($CDCl_3$) $\delta$ = 8.20, 8.16, 7.48, 7.27 (6-, 2-, 4-, 5-H des Pyridinteils), 7.20-6.90 (8 Aryl-H), 6.70 (C=C<u>H</u>), 5.90 (C<u>H</u>OH).

Analog zu diesen Beispielen können folgende Verbindungen hergestellt werden:

Tabelle

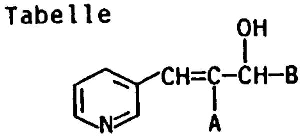

| Bsp.Nr. | A | B | Phys. Daten (Fp, IR, ¹H-NMR) |
|---|---|---|---|
| 1 | $CH_3$ | Phenyl | |
| 2 | $CH_3$ | 2-Methylphenyl | |
| 3 | $CH_3$ | 3-Methylphenyl | |
| 4 | $CH_3$ | 4-Methylphenyl | |
| 5 | $CH_3$ | 2,4-Dimethylphenyl | |
| 6 | $CH_3$ | 2,6-Dimethylphenyl | |
| 7 | $CH_3$ | 2,4,6-Trimethylphenyl | |
| 8 | $CH_3$ | 4-Ethylphenyl | |
| 9 | $CH_3$ | 4-Isopropylphenyl | |
| 10 | $CH_3$ | 4-tert.-Butylphenyl | |
| 11 | $CH_3$ | 1-Naphthyl | |
| 12 | $CH_3$ | 2-Naphthyl | |
| 13 | $CH_3$ | 4-Biphenyl | |
| 14 | $CH_3$ | 4-(2'-Chlorbiphenyl) | |
| 15 | $CH_3$ | 4-(4'-Chlorbiphenyl) | |
| 16 | $CH_3$ | 2-Methoxyphenyl | |
| 17 | $CH_3$ | 3-Methoxyphenyl | |
| 18 | $CH_3$ | 4-Methoxyphenyl | |
| 19 | $CH_3$ | 3,4-Dimethoxyphenyl | |

| Bsp.Nr. | A | B | Phys. Daten |
|---|---|---|---|
| 20 | $CH_3$ | 3,4,5-Trimethoxyphenyl | |
| 21 | $CH_3$ | 4-tert.-Butoxyphenyl | |
| 22 | $CH_3$ | 2-Trifluormethylphenyl | |
| 23 | $CH_3$ | 3-Trifluormethylphenyl | |
| 24 | $CH_3$ | 4-Trifluormethylphenyl | |
| 25 | $CH_3$ | 4-Difluormethoxyphenyl | |
| 26 | $CH_3$ | 4-Trifluormethoxyphenyl | |
| 27 | $CH_3$ | 4-Tetrafluorethoxyphenyl | |
| 28 | $CH_3$ | 2-Fluorphenyl | |
| 29 | $CH_3$ | 3-Fluorphenyl | |
| 30 | $CH_3$ | 4-Fluorphenyl | |
| 31 | $CH_3$ | 2,4-Difluorphenyl | |
| 32 | $CH_3$ | 2-Chlorphenyl | |
| 33 | $CH_3$ | 3-Chlorphenyl | |
| 34 | $CH_3$ | 4-Chlorphenyl | |
| 35 | $CH_3$ | 2,4-Dichlorphenyl | |
| 36 | $CH_3$ | 3,4-Dichlorphenyl | |
| 37 | $CH_3$ | 3,5-Dichlorphenyl | |
| 38 | $CH_3$ | 2,4,6-Trichlorphenyl | |
| 39 | $CH_3$ | 2-Chlor-4-fluorphenyl | |
| 40 | $CH_3$ | 4-Chlor-2-fluorphenyl | |
| 41 | $CH_3$ | 4-Bromphenyl | |
| 42 | $CH_3$ | Benzyl | |
| 43 | $CH_3$ | 2-Methylbenzyl | |
| 44 | $CH_3$ | 4-Methylbenzyl | |
| 45 | $CH_3$ | 2,4-Dimethylbenzyl | |

| Bsp.Nr. | A | B | Phys. Daten |
|---|---|---|---|
| 46 | $CH_3$ | 2-Fluorbenzyl | |
| 47 | $CH_3$ | 3-Fluorbenzyl | |
| 48 | $CH_3$ | 4-Fluorbenzyl | |
| 49 | $CH_3$ | 2-Chlorbenzyl | |
| 50 | $CH_3$ | 3-Chlorbenzyl | |
| 51 | $CH_3$ | 4-Chlorbenzyl | |
| 52 | $CH_3$ | 2,4-Dichlorbenzyl | |

| Bsp.Nr. | A | B | Phys. Daten |
|---|---|---|---|
| 66 | $C_2H_5$ | Phenyl | 3219, 3084, 3060, 2967, 2874, 1452, 1046, 1026, 702 |
| 67 | $C_2H_5$ | 2-Methylphenyl | |
| 68 | $C_2H_5$ | 3-Methylphenyl | |
| 69 | $C_2H_5$ | 4-Methylphenyl | |
| 70 | $C_2H_5$ | 2,4-Dimethylphenyl | Fp. 200-201°C |
| 71 | $C_2H_5$ | 2,6-Dimethylphenyl | 3227, 2964, 2933, 2874, 1468, 1412, 1076, 1027, 771, 713 |
| 72 | $C_2H_5$ | 2,4,6-Trimethylphenyl | Fp. 178-180°C |
| 73 | $C_2H_5$ | 4-Ethylphenyl | |
| 74 | $C_2H_5$ | 4-Isopropylphenyl | |
| 75 | $C_2H_5$ | 4-tert.-Butylphenyl | |
| 76 | $C_2H_5$ | 1-Naphthyl | |
| 77 | $C_2H_5$ | 2-Naphthyl | |
| 78 | $C_2H_5$ | 4-Biphenyl | 3205, 3055, 3029, 2971, 1486, 1408, 1369, 1265, 764, 700 |
| 79 | $C_2H_5$ | 4-(2'-Chlorbiphenyl) | |
| 80 | $C_2H_5$ | 4-(4'-Chlorbiphenyl) | |
| 81 | $C_2H_5$ | 2-Methoxyphenyl | |
| 82 | $C_2H_5$ | 3-Methoxyphenyl | |
| 83 | $C_2H_5$ | 4-Methoxyphenyl | |
| 84 | $C_2H_5$ | 3,4-Dimethoxyphenyl | |

| Bsp.Nr. | A | B | Phys. Daten |
|---|---|---|---|
| 85 | $C_2H_5$ | 3,4,5-Trimethoxyphenyl | |
| 86 | $C_2H_5$ | 4-tert.-Butoxyphenyl | |
| 87 | $C_2H_5$ | 2-Trifluormethylphenyl | |
| 88 | $C_2H_5$ | 3-Trifluormethylphenyl | |
| 89 | $C_2H_5$ | 4-Trifluormethylphenyl | |
| 90 | $C_2H_5$ | 4-Difluormethoxyphenyl | |
| 91 | $C_2H_5$ | 4-Trifluormethoxyphenyl | |
| 92 | $C_2H_5$ | 4-Tetrafluorethoxyphenyl | |
| 93 | $C_2H_5$ | 2-Fluorphenyl | |
| 94 | $C_2H_5$ | 3-Fluorphenyl | |
| 95 | $C_2H_5$ | 4-Fluorphenyl | |
| 96 | $C_2H_5$ | 2,4-Difluorphenyl | |
| 97 | $C_2H_5$ | 2-Chlorphenyl | |
| 98 | $C_2H_5$ | 3-Chlorphenyl | |
| 99 | $C_2H_5$ | 4-Chlorphenyl | Fp. 185°C |
| 100 | $C_2H_5$ | 2,4-Dichlorphenyl | |
| 101 | $C_2H_5$ | 3,4-Dichlorphenyl | |
| 102 | $C_2H_5$ | 3,5-Dichlorphenyl | |
| 103 | $C_2H_5$ | 2,4,6-Trichlorphenyl | |
| 104 | $C_2H_5$ | 2-Chlor-4-fluorphenyl | |
| 105 | $C_2H_5$ | 4-Chlor-2-fluorphenyl | |
| 106 | $C_2H_5$ | 4-Bromphenyl | |
| 107 | $C_2H_5$ | Benzyl | |
| 108 | $C_2H_5$ | 2-Methylbenzyl | |
| 109 | $C_2H_5$ | 4-Methylbenzyl | |
| 110 | $C_2H_5$ | 2,4-Dimethylbenzyl | |

| Bsp.Nr. | A | B | Phys. Daten |
|---|---|---|---|
| 111 | $C_2H_5$ | 2-Fluorbenzyl | |
| 112 | $C_2H_5$ | 3-Fluorbenzyl | |
| 113 | $C_2H_5$ | 4-Fluorbenzyl | |
| 114 | $C_2H_5$ | 2-Chlorbenzyl | |
| 115 | $C_2H_5$ | 3-Chlorbenzyl | |
| 116 | $C_2H_5$ | 4-Chlorbenzyl | |
| 117 | $C_2H_5$ | 2,4-Dichlorbenzyl | |
| 118 | $C_2H_5$ | 2-Chlor-4-fluorbenzyl | |

| Bsp.Nr. | A | B | Phys. Daten |
|---|---|---|---|
| 131 | n-$C_3H_7$ | Phenyl | 3217, 3083, 3060, 2958, 2930, 2870, 1452, 1027, 701 |
| 132 | n-$C_3H_7$ | 2-Methylphenyl | 3218, 2958, 2929, 2870, 1478, 1462, 1413, 1028, 744, 713 |
| 133 | n-$C_3H_7$ | 3-Methylphenyl | |
| 134 | n-$C_3H_7$ | 4-Methylphenyl | 3219, 3023, 2958, 2928, 2869, 1454, 1413, 1043, 819, 713 |
| 135 | n-$C_3H_7$ | 2,4-Dimethylphenyl | 3223, 2958, 2927, 2870, 1475, 1414, 1028, 713 |
| 136 | n-$C_3H_7$ | 2,6-Dimethylphenyl | 3220, 2959, 2930, 2871, 1468, 1413, 1268, 1047, 1028, 771, 713 |
| 137 | n-$C_3H_7$ | 2,4,6-Trimethylphenyl | 3221, 2958, 2928, 2870, 1477, 1464, 1045, 1028, 851, 714 |
| 138 | n-$C_3H_7$ | 4-Ethylphenyl | |
| 139 | n-$C_3H_7$ | 4-Isopropylphenyl | |
| 140 | n-$C_3H_7$ | 4-tert.-Butylphenyl | |
| 141 | n-$C_3H_7$ | 1-Naphthyl | |
| 142 | n-$C_3H_7$ | 2-Naphthyl | |
| 143 | n-$C_3H_7$ | 4-Biphenyl | 3200, 3027, 2958, 2930, 2869, 1486, 1044, 767, 746, 713, 693 |
| 144 | n-$C_3H_7$ | 4-(2'-Chlorbiphenyl) | |
| 145 | n-$C_3H_7$ | 4-(4'-Chlorbiphenyl) | |
| 146 | n-$C_3H_7$ | 2-Methoxyphenyl | |
| 147 | n-$C_3H_7$ | 3-Methoxyphenyl | |
| 148 | n-$C_3H_7$ | 4-Methoxyphenyl | |
| 149 | n-$C_3H_7$ | 3,4-Dimethoxyphenyl | |

| Bsp.Nr. | A | B | Phys. Daten |
|---|---|---|---|
| 150 | n-$C_3H_7$ | 3,4,5-Trimethoxyphenyl | |
| 151 | n-$C_3H_7$ | 4-tert.-Butoxyphenyl | |
| 152 | n-$C_3H_7$ | 2-Trifluormethylphenyl | |
| 153 | n-$C_3H_7$ | 3-Trifluormethylphenyl | |
| 154 | n-$C_3H_7$ | 4-Trifluormethylphenyl | |
| 155 | n-$C_3H_7$ | 4-Difluormethoxyphenyl | |
| 156 | n-$C_3H_7$ | 4-Trifluormethoxyphenyl | |
| 157 | n-$C_3H_7$ | 4-Tetrafluorethoxyphenyl | |
| 158 | n-$C_3H_7$ | 2-Fluorphenyl | |
| 159 | n-$C_3H_7$ | 3-Fluorphenyl | |
| 160 | n-$C_3H_7$ | 4-Fluorphenyl | 2959, 2871, 1602, 1506, 1221, 1155, 1027, 838, 712 |
| 161 | n-$C_3H_7$ | 2,4-Difluorphenyl | |
| 162 | n-$C_3H_7$ | 2-Chlorphenyl | |
| 163 | n-$C_3H_7$ | 3-Chlorphenyl | |
| 164 | n-$C_3H_7$ | 4-Chlorphenyl | 3203, 2959, 2930, 2870, 1487, 1412, 1090, 1014, 828, 712<br>Fp. 166-167°C (HCl-Salz) |
| 165 | n-$C_3H_7$ | 2,4-Dichlorphenyl | |
| 166 | n-$C_3H_7$ | 3,4-Dichlorphenyl | |
| 167 | n-$C_3H_7$ | 3,5-Dichlorphenyl | |
| 168 | n-$C_3H_7$ | 2,4,6-Trichlorphenyl | |
| 169 | n-$C_3H_7$ | 2-Chlor-4-fluorphenyl | |
| 170 | n-$C_3H_7$ | 4-Chlor-2-fluorphenyl | |
| 171 | n-$C_3H_7$ | 4-Bromphenyl | |
| 172 | n-$C_3H_7$ | Benzyl | |
| 173 | n-$C_3H_7$ | 2-Methylbenzyl | |

| Bsp.Nr. | A | B | Phys. Daten |
|---|---|---|---|
| 174 | n-$C_3H_7$ | 4-Methylbenzyl | |
| 175 | n-$C_3H_7$ | 2,4-Dimethylbenzyl | |
| 176 | n-$C_3H_7$ | 2-Fluorbenzyl | |
| 177 | n-$C_3H_7$ | 3-Fluorbenzyl | |
| 178 | n-$C_3H_7$ | 4-Fluorbenzyl | |
| 179 | n-$C_3H_7$ | 2-Chlorbenzyl | |
| 180 | n-$C_3H_7$ | 3-Chlorbenzyl | |
| 181 | n-$C_3H_7$ | 4-Chlorbenzyl | |
| 182 | n-$C_3H_7$ | 2,4-Dichlorbenzyl | |
| 183 | n-$C_3H_7$ | 2-Chlor-4-fluorbenzyl | |

| Bsp.Nr. | A | B | Phys. Daten |
|---|---|---|---|
| 196 | iso-$C_3H_7$ | Phenyl | |
| 197 | iso-$C_3H_7$ | 2-Methylphenyl | |
| 198 | iso-$C_3H_7$ | 3-Methylphenyl | |
| 199 | iso-$C_3H_7$ | 4-Methylphenyl | |
| 200 | iso-$C_3H_7$ | 2,4-Dimethylphenyl | |
| 201 | iso-$C_3H_7$ | 2,6-Dimethylphenyl | |
| 202 | iso-$C_3H_7$ | 2,4,6-Trimethylphenyl | |
| 203 | iso-$C_3H_7$ | 4-Ethylphenyl | |
| 204 | iso-$C_3H_7$ | 4-Isopropylphenyl | |
| 205 | iso-$C_3H_7$ | 4-tert.-Butylphenyl | |
| 206 | iso-$C_3H_7$ | 1-Naphthyl | |
| 207 | iso-$C_3H_7$ | 2-Naphthyl | |
| 208 | iso-$C_3H_7$ | 4-Biphenyl | |
| 209 | iso-$C_3H_7$ | 4-(2'-Chlorbiphenyl) | |
| 210 | iso-$C_3H_7$ | 4-(4'-Chlorbiphenyl) | |
| 211 | iso-$C_3H_7$ | 2-Methoxyphenyl | |
| 212 | iso-$C_3H_7$ | 3-Methoxyphenyl | |
| 213 | iso-$C_3H_7$ | 4-Methoxyphenyl | |
| 214 | iso-$C_3H_7$ | 3,4-Dimethoxyphenyl | |
| 215 | iso-$C_3H_7$ | 3,4,5-Trimethoxyphenyl | |
| 216 | iso-$C_3H_7$ | 4-tert.-Butoxyphenyl | |
| 217 | iso-$C_3H_7$ | 2-Trifluormethylphenyl | |
| 218 | iso-$C_3H_7$ | 3-Trifluormethylphenyl | |
| 219 | iso-$C_3H_7$ | 4-Trifluormethylphenyl | |
| 220 | iso-$C_3H_7$ | 4-Difluormethoxyphenyl | |
| 221 | iso-$C_3H_7$ | 4-Trifluormethoxyphenyl | |

| Bsp.Nr. | A | B | Phys. Daten |
|---|---|---|---|
| 222 | iso-$C_3H_7$ | 4-Tetrafluorethoxyphenyl | |
| 223 | iso-$C_3H_7$ | 2-Fluorphenyl | |
| 224 | iso-$C_3H_7$ | 3-Fluorphenyl | |
| 225 | iso-$C_3H_7$ | 4-Fluorphenyl | |
| 226 | iso-$C_3H_7$ | 2,4-Difluorphenyl | |
| 227 | iso-$C_3H_7$ | 2-Chlorphenyl | |
| 228 | iso-$C_3H_7$ | 3-Chlorphenyl | |
| 229 | iso-$C_3H_7$ | 4-Chlorphenyl | |
| 230 | iso-$C_3H_7$ | 2,4-Dichlorphenyl | |
| 231 | iso-$C_3H_7$ | 3,4-Dichlorphenyl | |
| 232 | iso-$C_3H_7$ | 3,5-Dichlorphenyl | |
| 233 | iso-$C_3H_7$ | 2,4,6-Trichlorphenyl | |
| 234 | iso-$C_3H_7$ | 2-Chlor-4-fluorphenyl | |
| 235 | iso-$C_3H_7$ | 4-Chlor-2-fluorphenyl | |
| 236 | iso-$C_3H_7$ | 4-Bromphenyl | |
| 237 | iso-$C_3H_7$ | Benzyl | |
| 238 | iso-$C_3H_7$ | 2-Methylbenzyl | |
| 239 | iso-$C_3H_7$ | 4-Methylbenzyl | |
| 240 | iso-$C_3H_7$ | 2,4-Dimethylbenzyl | |
| 241 | iso-$C_3H_7$ | 2-Fluorbenzyl | |
| 242 | iso-$C_3H_7$ | 3-Fluorbenzyl | |
| 243 | iso-$C_3H_7$ | 4-Fluorbenzyl | |
| 244 | iso-$C_3H_7$ | 2-Chlorbenzyl | |

| Bsp.Nr. | A | B | Phys. Daten |
|---|---|---|---|
| 245 | iso-$C_3H_7$ | 3-Chlorbenzyl | |
| 246 | iso-$C_3H_7$ | 4-Chlorbenzyl | |
| 247 | iso-$C_3H_7$ | 2,4-Dichlorbenzyl | |
| 248 | iso-$C_3H_7$ | 2-Chlor-4-fluorbenzyl | |

| Bsp.Nr. | A | B | Phys. Daten |
|---|---|---|---|
| 261 | n-$C_4H_9$ | Phenyl | 3208, 3028, 2956, 2931, 2870, 1453, 1413, 1025, 702 |
| 262 | n-$C_4H_9$ | 2-Methylphenyl | 3234, 2955, 2929, 2870, 1478, 1461, 1027, 753, 713 |
| 263 | n-$C_4H_9$ | 3-Methylphenyl | |
| 264 | n-$C_4H_9$ | 4-Methylphenyl | 3225, 2956, 2929, 2870, 1466, 1458, 1413, 1028, 820, 714 |
| 265 | n-$C_4H_9$ | 2,4-Dimethylphenyl | 3226, 3031, 2956, 2928, 2870, 1476, 1458, 1413, 1027, 713 |
| 266 | n-$C_4H_9$ | 2,6-Dimethylphenyl | |
| 267 | n-$C_4H_9$ | 2,4,6-Trimethylphenyl | |
| 268 | n-$C_4H_9$ | 4-Ethylphenyl | |
| 269 | n-$C_4H_9$ | 4-Isopropylphenyl | |
| 270 | n-$C_4H_9$ | 4-tert.-Butylphenyl | |
| 271 | n-$C_4H_9$ | 1-Naphthyl | |
| 272 | n-$C_4H_9$ | 2-Naphthyl | |
| 273 | n-$C_4H_9$ | 4-Biphenyl | $^1$H-NMR(E-Isomer) $\delta$ =6.80(C=CH) 5.38(CHOH) |
| 274 | n-$C_4H_9$ | 4-(2'-Chlorbiphenyl) | |
| 275 | n-$C_4H_9$ | 4-(4'-Chlorbiphenyl) | |
| 276 | n-$C_4H_9$ | 2-Methoxyphenyl | |
| 277 | n-$C_4H_9$ | 3-Methoxyphenyl | |
| 278 | n-$C_4H_9$ | 4-Methoxyphenyl | |
| 279 | n-$C_4H_9$ | 3,4-Dimethoxyphenyl | |

| Bsp.Nr. | A | B | Phys. Daten |
|---|---|---|---|
| 280 | n-$C_4H_9$ | 3,4,5-Trimethoxyphenyl | |
| 281 | n-$C_4H_9$ | 4-tert.-Butoxyphenyl | |
| 282 | n-$C_4H_9$ | 2-Trifluormethylphenyl | |
| 283 | n-$C_4H_9$ | 3-Trifluormethylphenyl | |
| 284 | n-$C_4H_9$ | 4-Trifluormethylphenyl | |
| 285 | n-$C_4H_9$ | 4-Difluormethoxyphenyl | |
| 286 | n-$C_4H_9$ | 4-Trifluormethoxyphenyl | |
| 287 | n-$C_4H_9$ | 4-Tetrafluorethoxyphenyl | |
| 288 | n-$C_4H_9$ | 2-Fluorphenyl | |
| 289 | n-$C_4H_9$ | 3-Fluorphenyl | |
| 290 | n-$C_4H_9$ | 4-Fluorphenyl | 3200,2957,2931,2870,1603,1507,1222, 1155,1028,838,713 |
| 291 | n-$C_4H_9$ | 2,4-Difluorphenyl | |
| 292 | n-$C_4H_9$ | 2-Chlorphenyl | |
| 293 | n-$C_4H_9$ | 3-Chlorphenyl | |
| 294 | n-$C_4H_9$ | 4-Chlorphenyl | 3343,2930,2869,1538,1488,1089,1015, 842,802,685 |
| 295 | n-$C_4H_9$ | 2,4-Dichlorphenyl | $^1$H-NMR(E-Isomer) $\delta$ = 6.58(C=CH), 5.70(CHOH) |
| 296 | n-$C_4H_9$ | 3,4-Dichlorphenyl | |
| 297 | n-$C_4H_9$ | 3,5-Dichlorphenyl | |
| 298 | n-$C_4H_9$ | 2,4,6-Trichlorphenyl | |
| 299 | n-$C_4H_9$ | 2-Chlor-4-fluorphenyl | |
| 300 | n-$C_4H_9$ | 4-Chlor-2-fluorphenyl | |
| 301 | n-$C_4H_9$ | 4-Bromphenyl | |
| 302 | n-$C_4H_9$ | Benzyl | |

| Bsp.Nr. | A | B | Phys. Daten |
|---|---|---|---|
| 303 | n-$C_4H_9$ | 2-Methylbenzyl | |
| 304 | n-$C_4H_9$ | 4-Methylbenzyl | |
| 305 | n-$C_4H_9$ | 2,4-Dimethylbenzyl | |
| 306 | n-$C_4H_9$ | 2-Fluorbenzyl | |
| 307 | n-$C_4H_9$ | 3-Fluorbenzyl | |
| 308 | n-$C_4H_9$ | 4-Fluorbenzyl | |
| 309 | n-$C_4H_9$ | 2-Chlorbenzyl | |
| 310 | n-$C_4H_9$ | 3-Chlorbenzyl | |
| 311 | n-$C_4H_9$ | 4-Chlorbenzyl | |
| 312 | n-$C_4H_9$ | 2,4-Dichlorbenzyl | |
| 313 | n-$C_4H_9$ | 2-Chlor-4-fluorbenzyl | |

| Bsp.Nr. | A | B | Phys. Daten |
|---|---|---|---|
| 326 | tert.-$C_4H_9$ | Phenyl | 3206, 2963, 2907, 2871, 1478, 1448, 1366, 1045, 1029, 705 |
| 327 | tert.-$C_4H_9$ | 2-Methylphenyl | |
| 328 | tert.-$C_4H_9$ | 3-Methylphenyl | |
| 329 | tert.-$C_4H_9$ | 4-Methylphenyl | |
| 330 | tert.-$C_4H_9$ | 2,4-Dimethylphenyl | |
| 331 | tert.-$C_4H_9$ | 2,6-Dimethylphenyl | |
| 332 | tert.-$C_4H_9$ | 2,4,6-Trimethylphenyl | |
| 333 | tert.-$C_4H_9$ | 4-Ethylphenyl | |
| 334 | tert.-$C_4H_9$ | 4-Isopropylphenyl | |
| 335 | tert.-$C_4H_9$ | 4-tert.-Butylphenyl | |
| 336 | tert.-$C_4H_9$ | 1-Naphthyl | |
| 337 | tert.-$C_4H_9$ | 2-Naphthyl | |
| 338 | tert.-$C_4H_9$ | 4-Biphenyl | |
| 339 | tert.-$C_4H_9$ | 4-(2'-Chlorbiphenyl) | |
| 340 | tert.-$C_4H_9$ | 4-(4'-Chlorbiphenyl) | |
| 341 | tert.-$C_4H_9$ | 2-Methoxyphenyl | |
| 342 | tert.-$C_4H_9$ | 3-Methoxyphenyl | |
| 343 | tert.-$C_4H_9$ | 4-Methoxyphenyl | |
| 344 | tert.-$C_4H_9$ | 3,4-Dimethoxyphenyl | |

| Bsp.Nr. | A | B | Phys. Daten |
|---|---|---|---|
| 345 | tert.-$C_4H_9$ | 3,4,5-Trimethoxyphenyl | |
| 346 | tert.-$C_4H_9$ | 4-tert.-Butoxyphenyl | |
| 347 | tert.-$C_4H_9$ | 2-Trifluormethylphenyl | |
| 348 | tert.-$C_4H_9$ | 3-Trifluormethylphenyl | |
| 349 | tert.-$C_4H_9$ | 4-Trifluormethylphenyl | |
| 350 | tert.-$C_4H_9$ | 4-Difluormethoxyphenyl | |
| 351 | tert.-$C_4H_9$ | 4-Trifluormethoxyphenyl | |
| 352 | tert.-$C_4H_9$ | 4-Tetrafluorethoxyphenyl | |
| 353 | tert.-$C_4H_9$ | 2-Fluorphenyl | |
| 354 | tert.-$C_4H_9$ | 3-Fluorphenyl | |
| 355 | tert.-$C_4H_9$ | 4-Fluorphenyl | 3223,1506,1485,1219,1028,825,812, 714 |
| 356 | tert.-$C_4H_9$ | 2,4-Difluorphenyl | |
| 357 | tert.-$C_4H_9$ | 2-Chlorphenyl | |
| 358 | tert.-$C_4H_9$ | 3-Chlorphenyl | |
| 359 | tert.-$C_4H_9$ | 4-Chlorphenyl | 3180,2970,2962,1483,1479,1394,1065, 1027,1003,804,750,714 |
| 360 | tert.-$C_4H_9$ | 2,4-Dichlorphenyl | 3200,2967,2908,2871,1467,1381,1091, 1066,1037,713 |
| 361 | tert.-$C_4H_9$ | 3,4-Dichlorphenyl | |
| 362 | tert.-$C_4H_9$ | 3,5-Dichlorphenyl | |
| 363 | tert.-$C_4H_9$ | 2,4,6-Trichlorphenyl | |
| 364 | tert.-$C_4H_9$ | 2-Chlor-4-fluorphenyl | |
| 365 | tert.-$C_4H_9$ | 4-Chlor-2-fluorphenyl | |
| 366 | tert.-$C_4H_9$ | 4-Bromphenyl | |
| 367 | tert.-$C_4H_9$ | Benzyl | |

| Bsp.Nr. | A | B | Phys. Daten |
|---|---|---|---|
| 368 | tert.-$C_4H_9$ | 2-Methylbenzyl | |
| 369 | tert.-$C_4H_9$ | 4-Methylbenzyl | |
| 370 | tert.-$C_4H_9$ | 2,4-Dimethylbenzyl | |
| 371 | tert.-$C_4H_9$ | 2-Fluorbenzyl | |
| 372 | tert.-$C_4H_9$ | 3-Fluorbenzyl | |
| 373 | tert.-$C_4H_9$ | 4-Fluorbenzyl | |
| 374 | tert.-$C_4H_9$ | 2-Chlorbenzyl | |
| 375 | tert.-$C_4H_9$ | 3-Chlorbenzyl | |
| 376 | tert.-$C_4H_9$ | 4-Chlorbenzyl | |
| 377 | tert.-$C_4H_9$ | 2,4-Dichlorbenzyl | |
| 378 | tert.-$C_4H_9$ | 2-Chlor-4-fluorbenzyl | |

| Bsp.Nr. | A | B | Phys. Daten |
|---|---|---|---|
| 391 | n-$C_5H_{11}$ | Phenyl | 3240,2954,2927,2869,1453,1414,1026, 701 |
| 392 | n-$C_5H_{11}$ | 2-Methylphenyl | |
| 393 | n-$C_5H_{11}$ | 3-Methylphenyl | |
| 394 | n-$C_5H_{11}$ | 4-Methylphenyl | |
| 395 | n-$C_5H_{11}$ | 2,4-Dimethylphenyl | 3240,2954,2927,2927,2869,1466,1457, 1414,1028,713 |
| 396 | n-$C_5H_{11}$ | 2,6-Dimethylphenyl | |
| 397 | n-$C_5H_{11}$ | 2,4,6-Trimethylphenyl | |
| 398 | n-$C_5H_{11}$ | 4-Ethylphenyl | |
| 399 | n-$C_5H_{11}$ | 4-Isopropylphenyl | |
| 400 | n-$C_5H_{11}$ | 4-tert.-Butylphenyl | |
| 401 | n-$C_5H_{11}$ | 1-Naphthyl | |
| 402 | n-$C_5H_{11}$ | 2-Naphthyl | |
| 403 | n-$C_5H_{11}$ | 4-Biphenyl | |
| 404 | n-$C_5H_{11}$ | 4-(2'-Chlorbiphenyl) | |
| 405 | n-$C_5H_{11}$ | 4-(4'-Chlorbiphenyl) | |
| 406 | n-$C_5H_{11}$ | 2-Methoxyphenyl | |
| 407 | n-$C_5H_{11}$ | 3-Methoxyphenyl | |
| 408 | n-$C_5H_{11}$ | 4-Methoxyphenyl | |
| 409 | n-$C_5H_{11}$ | 3,4-Dimethoxyphenyl | |

| Bsp.Nr. | A | B | Phys. Daten |
|---|---|---|---|
| 410 | n-$C_5H_{11}$ | 3,4,5-Trimethoxyphenyl | |
| 411 | n-$C_5H_{11}$ | 4-tert.-Butoxyphenyl | |
| 412 | n-$C_5H_{11}$ | 2-Trifluormethylphenyl | |
| 413 | n-$C_5H_{11}$ | 3-Trifluormethylphenyl | |
| 414 | n-$C_5H_{11}$ | 4-Trifluormethylphenyl | |
| 415 | n-$C_5H_{11}$ | 4-Difluormethoxyphenyl | |
| 416 | n-$C_5H_{11}$ | 4-Trifluormethoxyphenyl | |
| 417 | n-$C_5H_{11}$ | 4-Tetrafluorethoxyphenyl | |
| 418 | n-$C_5H_{11}$ | 2-Fluorphenyl | |
| 419 | n-$C_5H_{11}$ | 3-Fluorphenyl | |
| 420 | n-$C_5H_{11}$ | 4-Fluorphenyl | 3200, 2955, 2930, 2869, 1603, 1507, 1227 1155, 838, 713 |
| 421 | n-$C_5H_{11}$ | 2,4-Difluorphenyl | |
| 422 | n-$C_5H_{11}$ | 2-Chlorphenyl | |
| 423 | n-$C_5H_{11}$ | 3-Chlorphenyl | |
| 424 | n-$C_5H_{11}$ | 4-Chlorphenyl | 3208, 2955, 2929, 2869, 1488, 1090, 1015, 827, 712 |
| 425 | n-$C_5H_{11}$ | 2,4-Dichlorphenyl | Fp. 153-155°C (x HCl) |
| 426 | n-$C_5H_{11}$ | 3,4-Dichlorphenyl | |
| 427 | n-$C_5H_{11}$ | 3,5-Dichlorphenyl | |
| 428 | n-$C_5H_{11}$ | 2,4,6-Trichlorphenyl | |
| 429 | n-$C_5H_{11}$ | 2-Chlor-4-fluorphenyl | |
| 430 | n-$C_5H_{11}$ | 4-Chlor-2-fluorphenyl | |
| 431 | n-$C_5H_{11}$ | 4-Bromphenyl | |
| 432 | n-$C_5H_{11}$ | Benzyl | |

| Bsp.Nr. | A | B | Phys. Daten |
|---|---|---|---|
| 433 | n-$C_5H_{11}$ | 2-Methylbenzyl | |
| 434 | n-$C_5H_{11}$ | 4-Methylbenzyl | |
| 435 | n-$C_5H_{11}$ | 2,4-Dimethylbenzyl | |
| 436 | n-$C_5H_{11}$ | 2-Fluorbenzyl | |
| 437 | n-$C_5H_{11}$ | 3-Fluorbenzyl | |
| 438 | n-$C_5H_{11}$ | 4-Fluorbenzyl | |
| 439 | n-$C_5H_{11}$ | 2-Chlorbenzyl | |
| 440 | n-$C_5H_{11}$ | 3-Chlorbenzyl | |
| 441 | n-$C_5H_{11}$ | 4-Chlorbenzyl | |
| 442 | n-$C_5H_{11}$ | 2,4-Dichlorbenzyl | |
| 443 | n-$C_5H_{11}$ | 2-Chlor-4-fluorbenzyl | |

| Bsp.Nr. | A | B | Phys. Daten |
|---|---|---|---|
| 456 | neo-$C_5H_{11}$ | Phenyl | $^1$H-NMR: E-Isomer: $\delta$ =7.05 (C=CH), 5.38 (C$\underline{H}$OH), 2.58,1.63,0.80 ($CH_2C(CH_3)_3$). Z-Isomer: $\delta$ =6.62 (C=C$\underline{H}$), 5.70(C$\underline{H}$OH), 2.10,1.98,0.98 ($CH_2C(CH_3)_3$). |
| 457 | neo-$C_5H_{11}$ | 2-Methylphenyl | 2952,2864,1476,1464,1364,1046,1028, 760,728,715 |
| 458 | neo-$C_5H_{11}$ | 3-Methylphenyl | |
| 459 | neo-$C_5H_{11}$ | 4-Methylphenyl | Fp. 154-156°C (x HCl) |
| 460 | neo-$C_5H_{11}$ | 2,4-Dimethylphenyl | 3227,2951,2864,1476,1467,1412,1363, 1045,1028,715 |
| 461 | neo-$C_5H_{11}$ | 2,6-Dimethylphenyl | |
| 462 | neo-$C_5H_{11}$ | 2,4,6-Trimethylphenyl | |
| 463 | neo-$C_5H_{11}$ | 4-Ethylphenyl | |
| 464 | neo-$C_5H_{11}$ | 4-Isopropylphenyl | |
| 465 | neo-$C_5H_{11}$ | 4-tert.-Butylphenyl | |
| 466 | neo-$C_5H_{11}$ | 1-Naphthyl | |
| 467 | neo-$C_5H_{11}$ | 2-Naphthyl | |
| 468 | neo-$C_5H_{11}$ | 4-Biphenyl | |
| 469 | neo-$C_5H_{11}$ | 4-(2'-Chlorbiphenyl) | |
| 470 | neo-$C_5H_{11}$ | 4-(4'-Chlorbiphenyl) | |
| 471 | neo-$C_5H_{11}$ | 2-Methoxyphenyl | |
| 472 | neo-$C_5H_{11}$ | 3-Methoxyphenyl | |
| 473 | neo-$C_5H_{11}$ | 4-Methoxyphenyl | |
| 474 | neo-$C_5H_{11}$ | 3,4-Dimethoxyphenyl | |

| Bsp.Nr. | A | B | Phys. Daten |
|---|---|---|---|
| 475 | neo-$C_5H_{11}$ | 3,4,5-Trimethoxyphenyl | |
| 476 | neo-$C_5H_{11}$ | 4-tert.-Butoxyphenyl | |
| 477 | neo-$C_5H_{11}$ | 2-Trifluormethylphenyl | |
| 478 | neo-$C_5H_{11}$ | 3-Trifluormethylphenyl | |
| 479 | neo-$C_5H_{11}$ | 4-Trifluormethylphenyl | |
| 480 | neo-$C_5H_{11}$ | 4-Difluormethoxyphenyl | |
| 481 | neo-$C_5H_{11}$ | 4-Trifluormethoxyphenyl | |
| 482 | neo-$C_5H_{11}$ | 4-Tetrafluorethoxyphenyl | |
| 483 | neo-$C_5H_{11}$ | 2-Fluorphenyl | |
| 484 | neo-$C_5H_{11}$ | 3-Fluorphenyl | |
| 485 | neo-$C_5H_{11}$ | 4-Fluorphenyl | $^1H$-NMR: E-Isomer: $\delta$ =7.00 (C=C$\underline{H}$) 5.38 (C$\underline{H}$OH), 0.78 ($CMe_3$). Z-Isomer: $\delta$ =6.60 (C=C$\underline{H}$), 5.65 (C$\underline{H}$OH), 0.97 ($CMe_3$) |
| 486 | neo-$C_5H_{11}$ | 2,4-Difluorphenyl | |
| 487 | neo-$C_5H_{11}$ | 2-Chlorphenyl | |
| 488 | neo-$C_5H_{11}$ | 3-Chlorphenyl | |
| 489 | neo-$C_5H_{11}$ | 4-Chlorphenyl | $^1H$-NMR: E-Isomer: $\delta$ =7.00 (C=C$\underline{H}$), 5.35 (C$\underline{H}$OH), 2.55,1.63,0.78 ($CH_2CMe_3$). Z-Isomer: $\delta$ =6.58 (C=C$\underline{H}$), 5.62 (C$\underline{H}$OH), 2.08,1.95,0.98 ($CH_2CMe_3$) |
| 490 | neo-$C_5H_{11}$ | 2,4-Dichlorphenyl | Fp. 120°C(Zers.) (x HCl) |
| 491 | neo-$C_5H_{11}$ | 3,4-Dichlorphenyl | |
| 492 | neo-$C_5H_{11}$ | 3,5-Dichlorphenyl | |
| 493 | neo-$C_5H_{11}$ | 2,4,6-Trichlorphenyl | |
| 494 | neo-$C_5H_{11}$ | 2-Chlor-4-fluorphenyl | |

| Bsp.Nr. | A | B | Phys. Daten |
|---|---|---|---|
| 495 | neo-$C_5H_{11}$ | 4-Chlor-2-fluorphenyl | |
| 496 | neo-$C_5H_{11}$ | 4-Bromphenyl | |
| 497 | neo-$C_5H_{11}$ | Benzyl | |
| 498 | neo-$C_5H_{11}$ | 2-Methylbenzyl | |
| 499 | neo-$C_5H_{11}$ | 4-Methylbenzyl | |
| 500 | neo-$C_5H_{11}$ | 2,4-Dimethylbenzyl | |
| 501 | neo-$C_5H_{11}$ | 2-Fluorbenzyl | |
| 502 | neo-$C_5H_{11}$ | 3-Fluorbenzyl | |
| 503 | neo-$C_5H_{11}$ | 4-Fluorbenzyl | |
| 504 | neo-$C_5H_{11}$ | 2-Chlorbenzyl | |
| 505 | neo-$C_5H_{11}$ | 3-Chlorbenzyl | |
| 506 | neo-$C_5H_{11}$ | 4-Chlorbenzyl | |
| 507 | neo-$C_5H_{11}$ | 2,4-Dichlorbenzyl | |
| 508 | neo-$C_5H_{11}$ | 2-Chlor-4-fluorbenzyl | |

| Bsp.Nr. | A | B | Phys. Daten |
|---|---|---|---|
| 521 | n-$C_6H_{13}$ | Phenyl | 3219,2954,2927,2856,1467,1453,1045, 1026,713,701 |
| 522 | n-$C_6H_{13}$ | 2-Methylphenyl | |
| 523 | n-$C_6H_{13}$ | 3-Methylphenyl | |
| 524 | n-$C_6H_{13}$ | 4-Methylphenyl | |
| 525 | n-$C_6H_{13}$ | 2,4-Dimethylphenyl | |
| 526 | n-$C_6H_{13}$ | 2,6-Dimethylphenyl | |
| 527 | n-$C_6H_{13}$ | 2,4,6-Trimethylphenyl | |
| 528 | n-$C_6H_{13}$ | 4-Ethylphenyl | |
| 529 | n-$C_6H_{13}$ | 4-Isopropylphenyl | |
| 530 | n-$C_6H_{13}$ | 4-tert.-Butylphenyl | |
| 531 | n-$C_6H_{13}$ | 1-Naphthyl | |
| 532 | n-$C_6H_{13}$ | 2-Naphthyl | |
| 533 | n-$C_6H_{13}$ | 4-Biphenyl | |
| 534 | n-$C_6H_{13}$ | 4-(2'-Chlorbiphenyl) | |
| 535 | n-$C_6H_{13}$ | 4-(4'-Chlorbiphenyl) | |
| 536 | n-$C_6H_{13}$ | 2-Methoxyphenyl | |
| 537 | n-$C_6H_{13}$ | 3-Methoxyphenyl | |
| 538 | n-$C_6H_{13}$ | 4-Methoxyphenyl | |
| 539 | n-$C_6H_{13}$ | 3,4-Dimethoxyphenyl | |
| 540 | n-$C_6H_{13}$ | 3,4,5-Trimethoxyphenyl | |
| 541 | n-$C_6H_{13}$ | 4-tert.-Butoxyphenyl | |
| 542 | n-$C_6H_{13}$ | 2-Trifluormethylphenyl | |

| Bsp.Nr. | A | B | Phys. Daten |
|---|---|---|---|
| 543 | n-$C_6H_{13}$ | 3-Trifluormethylphenyl | |
| 544 | n-$C_6H_{13}$ | 4-Trifluormethylphenyl | |
| 545 | n-$C_6H_{13}$ | 4-Difluormethoxyphenyl | |
| 546 | n-$C_6H_{13}$ | 4-Trifluormethoxyphenyl | |
| 547 | n-$C_6H_{13}$ | 4-Tetrafluorethoxyphenyl | |
| 548 | n-$C_6H_{13}$ | 2-Fluorphenyl | |
| 549 | n-$C_6H_{13}$ | 3-Fluorphenyl | |
| 550 | n-$C_6H_{13}$ | 4-Fluorphenyl | 3200, 2955, 2929, 2857, 1507, 1222, 1155 838, 713 |
| 551 | n-$C_6H_{13}$ | 2,4-Difluorphenyl | |
| 552 | n-$C_6H_{13}$ | 2-Chlorphenyl | |
| 553 | n-$C_6H_{13}$ | 3-Chlorphenyl | |
| 554 | n-$C_6H_{13}$ | 4-Chlorphenyl | 3200, 2955, 2928, 2869, 2856, 1488, 1091, 1015, 829, 712 |
| 555 | n-$C_6H_{13}$ | 2,4-Dichlorphenyl | 3260, 2953, 2928, 2856, 1587, 1572, 1560, 1468, 1414, 1312, 715 |
| 556 | n-$C_6H_{13}$ | 3,4-Dichlorphenyl | |
| 557 | n-$C_6H_{13}$ | 3,5-Dichlorphenyl | |
| 558 | n-$C_6H_{13}$ | 2,4,6-Trichlorphenyl | |
| 559 | n-$C_6H_{13}$ | 2-Chlor-4-fluorphenyl | |
| 560 | n-$C_6H_{13}$ | 4-Chlor-2-fluorphenyl | |
| 561 | n-$C_6H_{13}$ | 4-Bromphenyl | |
| 562 | n-$C_6H_{13}$ | Benzyl | |

Herstellung und ihre Verwendung als Fungizide

| Bsp.Nr. | A | B | Phys. Daten |
|---|---|---|---|
| 563 | n-$C_6H_{13}$ | 2-Methylbenzyl | |
| 564 | n-$C_6H_{13}$ | 4-Methylbenzyl | |
| 565 | n-$C_6H_{13}$ | 2,4-Dimethylbenzyl | |
| 566 | n-$C_6H_{13}$ | 2-Fluorbenzyl | |
| 567 | n-$C_6H_{13}$ | 3-Fluorbenzyl | |
| 568 | n-$C_6H_{13}$ | 4-Fluorbenzyl | |
| 569 | n-$C_6H_{13}$ | 2-Chlorbenzyl | |
| 570 | n-$C_6H_{13}$ | 3-Chlorbenzyl | |
| 571 | n-$C_6H_{13}$ | 4-Chlorbenzyl | |
| 572 | n-$C_6H_{13}$ | 2,4-Dichlorbenzyl | |
| 573 | n-$C_6H_{13}$ | 2-Chlor-4-fluorbenzyl | |

| Bsp.Nr. | A | B | Phys. Daten |
|---|---|---|---|
| 586 | Phenyl | Phenyl | Fp. 149°C |
| 587 | Phenyl | 2-Methylphenyl | |
| 588 | Phenyl | 3-Methylphenyl | |
| 589 | Phenyl | 4-Methylphenyl | |
| 590 | Phenyl | 2,4-Dimethylphenyl | |
| 591 | Phenyl | 2,6-Dimethylphenyl | |
| 592 | Phenyl | 2,4,6-Trimethylphenyl | |
| 593 | Phenyl | 4-Ethylphenyl | |
| 594 | Phenyl | 4-Isopropylphenyl | |
| 595 | Phenyl | 4-tert.-Butylphenyl | |
| 596 | Phenyl | 1-Naphthyl | |
| 597 | Phenyl | 2-Naphthyl | |
| 598 | Phenyl | 4-Biphenyl | |
| 599 | Phenyl | 4-(2'-Chlorbiphenyl) | |
| 600 | Phenyl | 4-(4'-Chlorbiphenyl) | |
| 601 | Phenyl | 2-Methoxyphenyl | |
| 602 | Phenyl | 3-Methoxyphenyl | |
| 603 | Phenyl | 4-Methoxyphenyl | |
| 604 | Phenyl | 3,4-Dimethoxyphenyl | |
| 605 | Phenyl | 3,4,5-Trimethoxyphenyl | |
| 606 | Phenyl | 4-tert.-Butoxyphenyl | |
| 607 | Phenyl | 2-Trifluormethylphenyl | |
| 608 | Phenyl | 3-Trifluormethylphenyl | |
| 609 | Phenyl | 4-Trifluormethylphenyl | |
| 610 | Phenyl | 4-Difluormethoxyphenyl | |
| 611 | Phenyl | 4-Trifluormethoxyphenyl | |

| Bsp.Nr. | A | B | Phys. Daten |
|---|---|---|---|
| 612 | Phenyl | 4-Tetrafluorethoxyphenyl | |
| 613 | Phenyl | 2-Fluorphenyl | |
| 614 | Phenyl | 3-Fluorphenyl | |
| 615 | Phenyl | 4-Fluorphenyl | |
| 616 | Phenyl | 2,4-Difluorphenyl | |
| 617 | Phenyl | 2-Chlorphenyl | |
| 618 | Phenyl | 3-Chlorphenyl | |
| 619 | Phenyl | 4-Chlorphenyl | Fp. 89°C |
| 620 | Phenyl | 2,4-Dichlorphenyl | |
| 621 | Phenyl | 3,4-Dichlorphenyl | |
| 622 | Phenyl | 3,5-Dichlorphenyl | |
| 623 | Phenyl | 2,4,6-Trichlorphenyl | |
| 624 | Phenyl | 2-Chlor-4-fluorphenyl | |
| 625 | Phenyl | 4-Chlor-2-fluorphenyl | |
| 626 | Phenyl | 4-Bromphenyl | |
| 627 | Phenyl | Benzyl | |
| 628 | Phenyl | 2-Methylbenzyl | |
| 629 | Phenyl | 4-Methylbenzyl | |
| 630 | Phenyl | 2,4-Dimethylbenzyl | |
| 631 | Phenyl | 2-Fluorbenzyl | |
| 632 | Phenyl | 3-Fluorbenzyl | |
| 633 | Phenyl | 4-Fluorbenzyl | |
| 634 | Phenyl | 2-Chlorbenzyl | |

| Bsp.Nr. | A | B | Phys. Daten |
|---|---|---|---|
| 635 | Phenyl | 3-Chlorbenzyl | |
| 636 | Phenyl | 4-Chlorbenzyl | |
| 637 | Phenyl | 2,4-Dichlorbenzyl | |
| 638 | Phenyl | 2-Chlor-4-fluorbenzyl | |

| Bsp.Nr. | A | B | Phys. Daten |
|---|---|---|---|
| 651 | 4-Fluorphenyl | Phenyl | Fp. 96°C |
| 652 | 4-Fluorphenyl | 2-Methylphenyl | |
| 653 | 4-Fluorphenyl | 3-Methylphenyl | |
| 654 | 4-Fluorphenyl | 4-Methylphenyl | |
| 655 | 4-Fluorphenyl | 2,4-Dimethylphenyl | |
| 656 | 4-Fluorphenyl | 2,6-Dimethylphenyl | |
| 657 | 4-Fluorphenyl | 2,4,6-Trimethylphenyl | |
| 658 | 4-Fluorphenyl | 4-Ethylphenyl | |
| 659 | 4-Fluorphenyl | 4-Isopropylphenyl | |
| 660 | 4-Fluorphenyl | 4-tert.-Butylphenyl | |
| 661 | 4-Fluorphenyl | 1-Naphthyl | |
| 662 | 4-Fluorphenyl | 2-Naphthyl | |
| 663 | 4-Fluorphenyl | 4-Biphenyl | |
| 664 | 4-Fluorphenyl | 4-(2'-Chlorbiphenyl) | |
| 665 | 4-Fluorphenyl | 4-(4'-Chlorbiphenyl) | |
| 666 | 4-Fluorphenyl | 2-Methoxyphenyl | |
| 667 | 4-Fluorphenyl | 3-Methoxyphenyl | |
| 668 | 4-Fluorphenyl | 4-Methoxyphenyl | |
| 669 | 4-Fluorphenyl | 3,4-Dimethoxyphenyl | |
| 670 | 4-Fluorphenyl | 3,4,5-Trimethoxyphenyl | |
| 671 | 4-Fluorphenyl | 4-tert.-Butoxyphenyl | |
| 672 | 4-Fluorphenyl | 2-Trifluormethylphenyl | |
| 673 | 4-Fluorphenyl | 3-Trifluormethylphenyl | |
| 674 | 4-Fluorphenyl | 4-Trifluormethylphenyl | |
| 675 | 4-Fluorphenyl | 4-Difluormethoxyphenyl | |
| 676 | 4-Fluorphenyl | 4-Trifluormethoxyphenyl | |

| Bsp.Nr. | A | B | Phys. Daten |
|---|---|---|---|
| 677 | 4-Fluorphenyl | 4-Tetrafluorethoxyphenyl | |
| 678 | 4-Fluorphenyl | 2-Fluorphenyl | |
| 679 | 4-Fluorphenyl | 3-Fluorphenyl | |
| 680 | 4-Fluorphenyl | 4-Fluorphenyl | |
| 681 | 4-Fluorphenyl | 2,4-Difluorphenyl | |
| 682 | 4-Fluorphenyl | 2-Chlorphenyl | Fp. 82-83°C |
| 683 | 4-Fluorphenyl | 3-Chlorphenyl | |
| 684 | 4-Fluorphenyl | 4-Chlorphenyl | Fp. 104°C |
| 685 | 4-Fluorphenyl | 2,4-Dichlorphenyl | |
| 686 | 4-Fluorphenyl | 3,4-Dichlorphenyl | |
| 687 | 4-Fluorphenyl | 3,5-Dichlorphenyl | |
| 688 | 4-Fluorphenyl | 2,4,6-Trichlorphenyl | |
| 689 | 4-Fluorphenyl | 2-Chlor-4-fluorphenyl | |
| 690 | 4-Fluorphenyl | 4-Chlor-2-fluorphenyl | |
| 691 | 4-Fluorphenyl | 4-Bromphenyl | |
| 692 | 4-Fluorphenyl | Benzyl | |
| 693 | 4-Fluorphenyl | 2-Methylbenzyl | |
| 694 | 4-Fluorphenyl | 4-Methylbenzyl | |
| 695 | 4-Fluorphenyl | 2,4-Dimethylbenzyl | |
| 696 | 4-Fluorphenyl | 2-Fluorbenzyl | |
| 697 | 4-Fluorphenyl | 3-Fluorbenzyl | |
| 698 | 4-Fluorphenyl | 4-Fluorbenzyl | |
| 699 | 4-Fluorphenyl | 2-Chlorbenzyl | |

| Bsp.Nr. | A | B | Phys. Daten |
|---|---|---|---|
| 700 | 4-Fluorphenyl | 3-Chlorbenzyl | |
| 701 | 4-Fluorphenyl | 4-Chlorbenzyl | |
| 702 | 4-Fluorphenyl | 2,4-Dichlorbenzyl | |
| 703 | 4-Fluorphenyl | 2-Chlor-4-fluorbenzyl | |

| Bsp.Nr. | A | B | Phys. Daten |
|---|---|---|---|
| 716 | 4-Chlorphenyl | Phenyl | |
| 717 | 4-Chlorphenyl | 2-Methylphenyl | |
| 718 | 4-Chlorphenyl | 3-Methylphenyl | |
| 719 | 4-Chlorphenyl | 4-Methylphenyl | |
| 720 | 4-Chlorphenyl | 2,4-Dimethylphenyl | |
| 721 | 4-Chlorphenyl | 2,6-Dimethylphenyl | |
| 722 | 4-Chlorphenyl | 2,4,6-Trimethylphenyl | |
| 723 | 4-Chlorphenyl | 4-Ethylphenyl | |
| 724 | 4-Chlorphenyl | 4-Isopropylphenyl | |
| 725 | 4-Chlorphenyl | 4-tert.-Butylphenyl | |
| 726 | 4-Chlorphenyl | 1-Naphthyl | |
| 727 | 4-Chlorphenyl | 2-Naphthyl | |
| 728 | 4-Chlorphenyl | 4-Biphenyl | |
| 729 | 4-Chlorphenyl | 4-(2'-Chlorbiphenyl) | |
| 730 | 4-Chlorphenyl | 4-(4'-Chlorbiphenyl) | |
| 731 | 4-Chlorphenyl | 2-Methoxyphenyl | |
| 732 | 4-Chlorphenyl | 3-Methoxyphenyl | |
| 733 | 4-Chlorphenyl | 4-Methoxyphenyl | |
| 734 | 4-Chlorphenyl | 3,4-Dimethoxyphenyl | |
| 735 | 4-Chlorphenyl | 3,4,5-Trimethoxyphenyl | |
| 736 | 4-Chlorphenyl | 4-tert.-Butoxyphenyl | |
| 737 | 4-Chlorphenyl | 2-Trifluormethylphenyl | |
| 738 | 4-Chlorphenyl | 3-Trifluormethylphenyl | |
| 739 | 4-Chlorphenyl | 4-Trifluormethylphenyl | |
| 740 | 4-Chlorphenyl | 4-Difluormethoxyphenyl | |
| 741 | 4-Chlorphenyl | 4-Trifluormethoxyphenyl | |

| Bsp.Nr. | A | B | Phys. Daten |
|---|---|---|---|
| 742 | 4-Chlorphenyl | 4-Tetrafluorethoxyphenyl | |
| 743 | 4-Chlorphenyl | 2-Fluorphenyl | |
| 744 | 4-Chlorphenyl | 3-Fluorphenyl | |
| 745 | 4-Chlorphenyl | 4-Fluorphenyl | |
| 746 | 4-Chlorphenyl | 2,4-Difluorphenyl | |
| 747 | 4-Chlorphenyl | 2-Chlorphenyl | |
| 748 | 4-Chlorphenyl | 3-Chlorphenyl | |
| 749 | 4-Chlorphenyl | 4-Chlorphenyl | |
| 750 | 4-Chlorphenyl | 2,4-Dichlorphenyl | |
| 751 | 4-Chlorphenyl | 3,4-Dichlorphenyl | |
| 752 | 4-Chlorphenyl | 3,5-Dichlorphenyl | |
| 753 | 4-Chlorphenyl | 2,4,6-Trichlorphenyl | |
| 754 | 4-Chlorphenyl | 2-Chlor-4-fluorphenyl | |
| 755 | 4-Chlorphenyl | 4-Chlor-2-fluorphenyl | |
| 756 | 4-Chlorphenyl | 4-Bromphenyl | |
| 757 | 4-Chlorphenyl | Benzyl | |
| 758 | 4-Chlorphenyl | 2-Methylbenzyl | |
| 759 | 4-Chlorphenyl | 4-Methylbenzyl | |
| 760 | 4-Chlorphenyl | 2,4-Dimethylbenzyl | |
| 761 | 4-Chlorphenyl | 2-Fluorbenzyl | |
| 762 | 4-Chlorphenyl | 3-Fluorbenzyl | |
| 763 | 4-Chlorphenyl | 4-Fluorbenzyl | |
| 764 | 4-Chlorphenyl | 2-Chlorbenzyl | |

| Bsp.Nr. | A | B | Phys. Daten |
|---|---|---|---|
| 765 | 4-Chlorphenyl | 3-Chlorbenzyl | |
| 766 | 4-Chlorphenyl | 4-Chlorbenzyl | |
| 767 | 4-Chlorphenyl | 2,4-Dichlorbenzyl | |
| 768 | 4-Chlorphenyl | 2-Chlor-4-fluorbenzyl | |

| Bsp.Nr. | A | B | Phys. Daten |
|---|---|---|---|
| 781 | 2,4-Dichlorphenyl | Phenyl | |
| 782 | 2,4-Dichlorphenyl | 2-Methylphenyl | |
| 783 | 2,4-Dichlorphenyl | 3-Methylphenyl | |
| 784 | 2,4-Dichlorphenyl | 4-Methylphenyl | |
| 785 | 2,4-Dichlorphenyl | 2,4-Dimethylphenyl | |
| 786 | 2,4-Dichlorphenyl | 2,6-Dimethylphenyl | |
| 787 | 2,4-Dichlorphenyl | 2,4,6-Trimethylphenyl | |
| 788 | 2,4-Dichlorphenyl | 4-Ethylphenyl | |
| 789 | 2,4-Dichlorphenyl | 4-Isopropylphenyl | |
| 790 | 2,4-Dichlorphenyl | 4-tert.-Butylphenyl | |
| 791 | 2,4-Dichlorphenyl | 1-Naphthyl | |
| 792 | 2,4-Dichlorphenyl | 2-Naphthyl | |
| 793 | 2,4-Dichlorphenyl | 4-Biphenyl | |
| 794 | 2,4-Dichlorphenyl | 4-(2'-Chlorbiphenyl) | |
| 795 | 2,4-Dichlorphenyl | 4-(4'-Chlorbiphenyl) | |
| 796 | 2,4-Dichlorphenyl | 2-Methoxyphenyl | |
| 797 | 2,4-Dichlorphenyl | 3-Methoxyphenyl | |
| 798 | 2,4-Dichlorphenyl | 4-Methoxyphenyl | |
| 799 | 2,4-Dichlorphenyl | 3,4-Dimethoxyphenyl | |
| 800 | 2,4-Dichlorphenyl | 3,4,5-Trimethoxyphenyl | |
| 801 | 2,4-Dichlorphenyl | 4-tert.-Butoxyphenyl | |
| 802 | 2,4-Dichlorphenyl | 2-Trifluormethylphenyl | |
| 803 | 2,4-Dichlorphenyl | 3-Trifluormethylphenyl | |
| 804 | 2,4-Dichlorphenyl | 4-Trifluormethylphenyl | |
| 805 | 2,4-Dichlorphenyl | 4-Difluormethoxyphenyl | |
| 806 | 2,4-Dichlorphenyl | 4-Trifluormethoxyphenyl | |

| Bsp.Nr. | A | B | Phys. Daten |
|---|---|---|---|
| 807 | 2,4-Dichlorphenyl | 4-Tetrafluorethoxyphenyl | |
| 808 | 2,4-Dichlorphenyl | 2-Fluorphenyl | |
| 809 | 2,4-Dichlorphenyl | 3-Fluorphenyl | |
| 810 | 2,4-Dichlorphenyl | 4-Fluorphenyl | |
| 811 | 2,4-Dichlorphenyl | 2,4-Difluorphenyl | |
| 812 | 2,4-Dichlorphenyl | 2-Chlorphenyl | |
| 813 | 2,4-Dichlorphenyl | 3-Chlorphenyl | |
| 814 | 2,4-Dichlorphenyl | 4-Chlorphenyl | |
| 815 | 2,4-Dichlorphenyl | 2,4-Dichlorphenyl | |
| 816 | 2,4-Dichlorphenyl | 3,4-Dichlorphenyl | |
| 817 | 2,4-Dichlorphenyl | 3,5-Dichlorphenyl | |
| 818 | 2,4-Dichlorphenyl | 2,4,6-Trichlorphenyl | |
| 819 | 2,4-Dichlorphenyl | 2-Chlor-4-fluorphenyl | |
| 820 | 2,4-Dichlorphenyl | 4-Chlor-2-fluorphenyl | |
| 821 | 2,4-Dichlorphenyl | 4-Bromphenyl | |
| 822 | 2,4-Dichlorphenyl | Benzyl | |
| 823 | 2,4-Dichlorphenyl | 2-Methylbenzyl | |
| 824 | 2,4-Dichlorphenyl | 4-Methylbenzyl | |
| 825 | 2,4-Dichlorphenyl | 2,4-Dimethylbenzyl | |
| 826 | 2,4-Dichlorphenyl | 2-Fluorbenzyl | |
| 827 | 2,4-Dichlorphenyl | 3-Fluorbenzyl | |
| 828 | 2,4-Dichlorphenyl | 4-Fluorbenzyl | |
| 829 | 2,4-Dichlorphenyl | 2-Chlorbenzyl | |

| Bsp.Nr. | A | B | Phys. Daten |
|---|---|---|---|
| 830 | 2,4-Dichlorphenyl | 3-Chlorbenzyl | |
| 831 | 2,4-Dichlorphenyl | 4-Chlorbenzyl | |
| 832 | 2,4-Dichlorphenyl | 2,4-Dichlorbenzyl | |
| 833 | 2,4-Dichlorphenyl | 2-Chlor-4-fluorbenzyl | |

| Bsp.Nr. | A | B | Phys. Daten |
|---|---|---|---|
| 846 | 4-Methoxyphenyl | Phenyl | |
| 847 | 4-Methoxyphenyl | 2-Methylphenyl | |
| 848 | 4-Methoxyphenyl | 3-Methylphenyl | |
| 849 | 4-Methoxyphenyl | 4-Methylphenyl | |
| 850 | 4-Methoxyphenyl | 2,4-Dimethylphenyl | |
| 851 | 4-Methoxyphenyl | 2,6-Dimethylphenyl | |
| 852 | 4-Methoxyphenyl | 2,4,6-Trimethylphenyl | |
| 853 | 4-Methoxyphenyl | 4-Ethylphenyl | |
| 854 | 4-Methoxyphenyl | 4-Isopropylphenyl | |
| 855 | 4-Methoxyphenyl | 4-tert.-Butylphenyl | |
| 856 | 4-Methoxyphenyl | 1-Naphthyl | |
| 857 | 4-Methoxyphenyl | 2-Naphthyl | |
| 858 | 4-Methoxyphenyl | 4-Biphenyl | |
| 859 | 4-Methoxyphenyl | 4-(2'-Chlorbiphenyl) | |
| 860 | 4-Methoxyphenyl | 4-(4'-Chlorbiphenyl) | |
| 861 | 4-Methoxyphenyl | 2-Methoxyphenyl | |
| 862 | 4-Methoxyphenyl | 3-Methoxyphenyl | |
| 863 | 4-Methoxyphenyl | 4-Methoxyphenyl | |
| 864 | 4-Methoxyphenyl | 3,4-Dimethoxyphenyl | |
| 865 | 4-Methoxyphenyl | 3,4,5-Trimethoxyphenyl | |
| 866 | 4-Methoxyphenyl | 4-tert.-Butoxyphenyl | |
| 867 | 4-Methoxyphenyl | 2-Trifluormethylphenyl | |
| 868 | 4-Methoxyphenyl | 3-Trifluormethylphenyl | |
| 869 | 4-Methoxyphenyl | 4-Trifluormethylphenyl | |
| 870 | 4-Methoxyphenyl | 4-Difluormethoxyphenyl | |
| 871 | 4-Methoxyphenyl | 4-Trifluormethoxyphenyl | |

| Bsp.Nr. | A | B | Phys. Daten |
|---|---|---|---|
| 872 | 4-Methoxyphenyl | 4-Tetrafluorethoxyphenyl | |
| 873 | 4-Methoxyphenyl | 2-Fluorphenyl | |
| 874 | 4-Methoxyphenyl | 3-Fluorphenyl | |
| 875 | 4-Methoxyphenyl | 4-Fluorphenyl | |
| 876 | 4-Methoxyphenyl | 2,4-Difluorphenyl | |
| 877 | 4-Methoxyphenyl | 2-Chlorphenyl | |
| 878 | 4-Methoxyphenyl | 3-Chlorphenyl | |
| 879 | 4-Methoxyphenyl | 4-Chlorphenyl | |
| 880 | 4-Methoxyphenyl | 2,4-Dichlorphenyl | |
| 881 | 4-Methoxyphenyl | 3,4-Dichlorphenyl | |
| 882 | 4-Methoxyphenyl | 3,5-Dichlorphenyl | |
| 883 | 4-Methoxyphenyl | 2,4,6-Trichlorphenyl | |
| 884 | 4-Methoxyphenyl | 2-Chlor-4-fluorphenyl | |
| 885 | 4-Methoxyphenyl | 4-Chlor-2-fluorphenyl | |
| 886 | 4-Methoxyphenyl | 4-Bromphenyl | |
| 887 | 4-Methoxyphenyl | Benzyl | |
| 888 | 4-Methoxyphenyl | 2-Methylbenzyl | |
| 889 | 4-Methoxyphenyl | 4-Methylbenzyl | |
| 890 | 4-Methoxyphenyl | 2,4-Dimethylbenzyl | |
| 891 | 4-Methoxyphenyl | 2-Fluorbenzyl | |
| 892 | 4-Methoxyphenyl | 3-Fluorbenzyl | |
| 893 | 4-Methoxyphenyl | 4-Fluorbenzyl | |
| 894 | 4-Methoxyphenyl | 2-Chlorbenzyl | |

| Bsp.Nr. | A | B | Phys. Daten |
|---|---|---|---|
| 895 | 4-Methoxyphenyl | 3-Chlorbenzyl | |
| 896 | 4-Methoxyphenyl | 4-Chlorbenzyl | |
| 897 | 4-Methoxyphenyl | 2,4-Dichlorbenzyl | |
| 898 | 4-Methoxyphenyl | 2-Chlor-4-fluorbenzyl | |

Die neuen Heteroarylalkene I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Besonders interessant sind sie für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse, wie Gurken, Bohnen und Kürbisgewächse.

Die neuen Heteroarylalkene sind insbesondere geeignet zur Bekämpfung folgender Schadpilze:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,

Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Heteroarylalkene werden angewendet, indem man die Pflanzen, Materialien oder Flächen mit den Wirkstoffen besprüht oder bestäubt oder die Samen der Pflanzen mit den Wirkstoffen behandelt. Die Anwendung erfolgt vor oder nach der Infektion durch die Pilze.

Die Heteroarylalkene können in die üblichen fungiziden Mittel (Formulierungen) übergeführt werden, d.h. in Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise und mit den üblichen Hilfsstoffen hergestellt, z.B. durch Verstrecken des Wirkstoffs mit Lösungsmitteln und/oder Trägerstoffen, gegebenenfalls unter Verwendung von Emulgiermitteln und Dispergiermitteln. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser (letzteres gegebenenfalls zusammen mit organischen Lösungsmitteln); Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95 Gew.-%, vorzugsweise zwischen 0,5 und 90 Gew.-% Wirkstoff. Die Aufwandmengen liegen je nach Art des gewünschten Effektes zwischen 0,02 und 3 kg Wirkstoff je ha.

Die Mittel werden, gegebenenfalls nach weiterer Verdünnung, in bekannter Weise angewendet, beispielsweise durch Versprühen, Vernebeln, Verstäuben, Verstreuen, Beizen oder Gießen.

Beispiele für Zubereitungen sind:

I. Man vermischt 90 Gew.-Teile der Verbindung Nr. 66 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 71 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 72 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 99 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 131 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 132 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 134 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 135 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 136 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäpen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene andere Fungizide, wie
Dodecylguanidinacetat,

Anwendungsbeispiele

Die fungizide Wirkung der Heteroarylalkene I wurde mit denen von 2-(2,4-Dichlorphenyl)-3-(3-pyridyl)-acrylsäure (A) - bekannt aus EP-A2-104 690 - und 1-(4-Chlorphenyl)-3-(3-pyridyl)-propen-2-on-1 (B) - bekannt unter CAS-Reg. Nr. 40665-19-8 - im Gewächshausversuch verglichen.

Beispiel A

Wirksamkeit gegen Pyrenophora teres

Die Versuche wurden mit den Wirkstoffen 66, 71, 72, 99, 100, 134, 135, 136, 137, 143, 262, 265, 273, 360, 490 und 555 durchgeführt.

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die

Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20-22°C und 70 % relativer Luftfeuchtigkeit für weitere 5 Tage kultiviert. Dann wurde das Ausmaß der Symptomentwicklung ermittelt.

Das Ergebnis zeigt, daß alle untersuchten Wirkstoffe bei Anwendung als 0,05 %ige Spritzaufbereitung eine bessere fungizide Wirkung zeigen (90 %) als der bekannte Vergleichswirkstoff A (40 %).

Beispiel B

Wirksamkeit gegen Gurkenmehltau

Die Versuche wurden mit den Wirkstoffen 295, 360, 490 und 555 durchgeführt.

Junge Gurkenpflanzen der Sorte "Chinesische Schlange" wurden im Zweiblattstadium mit einer wäßrigen Konidiensuspension des Gurkenmehltaus (Erysiphe cichoracearum und Sphaerotheca fuliginea) besprüht. Am nächsten Tag wurden diese Pflanzen mit wäßriger Spritzbrühe, die 80 % Wirkstoff und 20 % Emulgiermittel in der Trockensubstanz enthielt bis zur Tropfnäße besprüht und im Gewächshaus bei Temperaturen zwischen 20 und 22°C und 70 bis 80 % Luftfeuchtigkeit aufgestellt. 21 Tage nach der Wirkstoffapplikation wurde das Ausmaß des Pilzbefalls ermittelt.

Das Ergebnis zeigt, daß alle untersuchten Wirkstoffe bei Anwendung als 0,025 %ige wäßrige Wirkstoffaufbereitung eine bessere fungizide Wirksamkeit (95 %) zeigen als der bekannte Vergleichswirkstoff B (0 %).

Beispiel C

Wirksamkeit gegen Botrytis cinerea

Die Versuche wurden mit den Wirkstoffen 72, 136, 265, 273, 490 und 555 durchgeführt.

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß alle untersuchten Wirkstoffe bei Anwendung als 0,05 %ige wäßrige Wirkstoffaufbereitung eine bessere fungizide Wirksamkeit (90 %) zeigen als der bekannte Vergleichswirkstoff A (40 %).

## Patentansprüche

### Patentansprüche für folgende Vertragsstaaten : DE, GB, FR, IT, NL, SE, LI, CH, BE, AT, DK

**1.** Heteroarylalkene der allgemeinen Formel I,

```
Ar-CH=C-Z-B          I
      |
      A
```

in der die Substituenten folgende Bedeutung haben:

Ar 3-Pyridyl;

A $C_1$-$C_6$-Alkyl, $C_2$-$C_6$-Alkenyl, $C_3$-$C_8$-Cycloalkyl, ($C_3$-$C_8$)-Cycloalkyl-($C_1$-$C_3$)-alkyl, ein-, zwei- oder dreikerniges Aryl oder Aryl-$C_1$-$C_3$-alkyl, wobei Aryl jeweils ein-, zwei- oder dreifach durch die Reste $C_1$-$C_4$-Alkyl, $C_1$-$C_4$-Alkoxy, $C_1$-$C_4$-Mono-, Di- oder Trihalogenalkyl, $C_1$-$C_4$-Halogenalkoxy, Phenyl oder Halogen substituiert sein kann;

Z

```
 OH
 |
-CH-  .
```

B ein- oder zweikerniges Aryl oder Benzyl bedeutet, in denen die aromatischen Ringe jeweils ein-, zwei- oder dreifach durch $C_1$-$C_4$-Alkyl, $C_1$-$C_4$-Alkoxy, $C_1$-$C_4$-Halogenalkyl, $C_1$-$C_4$-Halogenalkoxy, Phenyl, Halogenphenyl oder Halogen substituiert sein können;

sowie deren N-Oxide und Säureadditionssalze mit anorganischen Mineralsäuren, Carbonsäuren oder einkernigen Arylsulfonsäuren.

2. Verfahren zur Herstellung von Heteroarylalkenen gemäß Anspruch 1, dadurch gekennzeichnet, daß man ein Heteroarylalkenal der allgemeinen Formel II

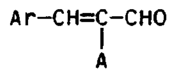

II

mit einer lithiumorganischen Verbindung Li-B oder einem Grignardreagenz BMgHal, wobei Hal für Halogen steht, zum Heteroarylalkenol der allgemeinen Formel Ia

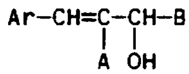

Ia

umsetzt, oder indem man einen Heteroarylaldehyd Ar-CHO mit einem Keton

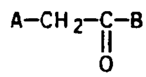

zum Heteroarylalkenon der allgemeinen Formel Ib

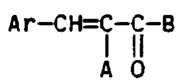

Ib

umsetzt und das Heteroarylalkenon der allgemeinen Formel Ib mit an sich bekannten Methoden zum Heteroarylalkenol der allgemeinen Formel Ia reduziert.

3. Heteroarylalkenale der allgemeinen Formel III,

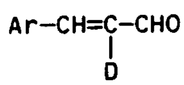

III

in der Ar 3-Pyridyl und

D für $C_2$-$C_6$-Alkyl, $C_3$-$C_8$-Cycloalkyl, ($C_3$-$C_8$-)-Cycloalkyl-($C_1$-$C_3$)-alkyl oder für ein- oder zweikerniges Aryl-($C_1$-$C_3$)alkyl steht, wobei Aryl ein-, zwei- oder dreifach durch die Reste $C_1$-$C_4$-Alkyl, $C_1$-$C_4$-Alkoxy, $C_1$-$C_4$-Halogenalkyl, $C_1$-$C_4$-Halogenalkoxy, Phenyl oder Halogen substituiert sein kann.

4. Verfahren zur Herstellung eines Heteroarylalkenals der allgemeinen Formel III gemäß Anspruch 3, dadurch gekennzeichnet, daß man zwei Aldehyde, D-$CH_2$CHO und Ar-CHO, in an sich bekannter Weise zum Heteroarylalkenal der allgemeinen Formel III umsetzt.

5. Fungizide Mittel, enthaltend ein Heteroarylalken, ein N-Oxid oder ein Säureadditionssalz gemäß Anspruch 1 und übliche Zusatzstoffe.

6. Fungizide Mittel gemäß Anspruch 5, enthaltend weitere wirksame Bestandteile.

7. Verfahren zur Bekämpfung von Schadpilzen, dadurch gekennzeichnet, daß man die Pilze oder die vom Pilzbefall bedrohten Pflanzen, Samen, Materialien oder Flächen mit einem Heteroarylalken I gemäß Anspruch 1 oder mit einem fungiziden Mittel nach den Ansprüchen 5 oder 6 behandelt.

**Patentansprüche für folgende Vertragsstaaten : GR, ES**

1. Verfahren zur Herstellung von Heteroarylalkenen der allgemeinen Formel I,

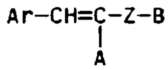

I

in der die Substituenten folgende Bedeutung haben:

Ar 3-Pyridyl;

A $C_1$-$C_6$-Alkyl, $C_2$-$C_6$-Alkenyl, $C_3$-$C_8$-Cycloalkyl, ($C_3$-$C_8$)-Cycloalkyl-($C_1$-$C_3$)-alkyl, ein-, zwei- oder dreikerniges Aryl oder Aryl-$C_1$-$C_3$-alkyl, wobei Aryl jeweils ein-, zwei- oder dreifach durch die Reste $C_1$-$C_4$-Alkyl, $C_1$-$C_4$-Alkoxy, $C_1$-$C_4$-Mono-, Di- oder Trihalogenalkyl, $C_1$-$C_4$-Halogenalkoxy, Phenyl oder Halogen substituiert sein kann;

Z

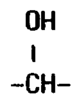

,

B ein- oder zweikerniges Aryl oder Benzyl bedeutet,
in denen die aromatischen Ringe jeweils ein-, zwei- oder dreifach durch $C_1$-$C_4$-Alkyl, $C_1$-$C_4$-Alkoxy, $C_1$-$C_4$-Halogenalkyl, $C_1$-$C_4$-Halogenalkoxy, Phenyl, Halogenphenyl oder Halogen substituiert sein können;

sowie deren N-Oxide und Säureadditionssalze mit anorganischen Mineralsäuren, Carbonsäuren oder einkernigen Arylsulfonsäuren, dadurch gekennzeichnet, daß man ein Heteroarylalkenal der allgemeinen Formel II

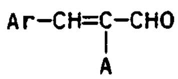

II

mit einer lithiumorganischen Verbindung Li-B oder einem Grignardreagenz BMgHal, wobei Hal für Halogen steht, zum Heteroarylalkenol der allgemeinen Formel Ia

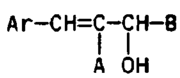

Ia

umsetzt, oder indem man einen Heteroarylaldehyd Ar-CHO mit einem Keton

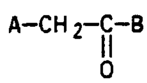

zum Heteroarylalkenon der allgemeinen Formel Ib

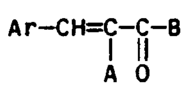

Ib

umsetzt und das Heteroarylalkenon der allgemeinen Formel Ib mit an sich bekannten Methoden zum Heteroarylalkenol der allgemeinen Formel Ia reduziert.

2. Verfahren zur Herstellung eines Heteroarylalkenals der allgemeinen Formel III

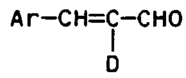 III

in der Ar 3-Pyridyl und
D für $C_2$-$C_6$-Alkyl, $C_3$-$C_8$-Cycloalkyl, ($C_3$-$C_8$-)-Cycloalkyl-($C_1$-$C_3$)-alkyl oder für ein- oder zweikerniges Aryl-($C_1$-$C_3$)alkyl steht, wobei Aryl ein-, zwei- oder dreifach durch die Reste $C_1$-$C_4$-Alkyl, $C_1$-$C_4$-Alkoxy, $C_1$-$C_4$-Halogenalkyl, $C_1$-$C_4$-Halogenalkoxy, Phenyl oder Halogen substituiert sein kann, dadurch gekennzeichnet, daß man zwei Aldehyde, D-$CH_2$CHO und Ar-CHO, in an sich bekannter Weise zum Heteroarylalkenal der allgemeinen Formel III umsetzt.

3. Fungizide Mittel, enthaltend ein Heteroarylalken, ein N-Oxid oder ein Säureadditionssalz gemäß Anspruch 1 und übliche Zusatzstoffe.

4. Fungizide Mittel gemäß Anspruch 3, enthaltend weitere wirksame Bestandteile.

5. Verfahren zur Bekämpfung von Schadpilzen, dadurch gekennzeichnet, daß man die Pilze oder die vom Pilzbefall bedrohten Pflanzen, Samen, Materialien oder Flächen mit einem Heteroarylalken I gemäß Anspruch 1 oder mit einem fungiziden Mittel nach den Ansprüchen 3 oder 4 behandelt.

## Claims

### Claims for the following Contracting States : DE, GB, FR, IT, NL, SE, LI, CH, BE, AT, DK

1. A hetarylalkene of the formula I

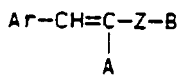 I

where
Ar is 3-pyridyl;
A is $C_1$-$C_6$-alkyl, $C_2$-$C_6$-alkenyl, $C_3$-$C_8$-cycloalkyl, $C_3$-$C_8$-cycloalkyl-$C_1$-$C_3$-alkyl or mononuclear, dinuclear or trinuclear aryl or aryl-$C_1$-$C_3$-alkyl, where aryl in each case may be monosubstituted, disubstituted or trisubstituted by $C_1$-$C_4$-alkyl, $C_1$-$C_4$-alkoxy, $C_1$-$C_4$-mono-, di- or trihaloalkyl, $C_1$-$C_4$-haloalkoxy, phenyl or halogen;
Z is

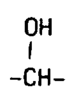 ;

B is mononuclear or dinuclear aryl or benzyl in which each of the aromatic rings may be monosubstituted, disubstituted or trisubstituted by $C_1$-$C_4$-alkyl, $C_1$-$C_4$-alkoxy, $C_1$-$C_4$-haloalkyl, $C_1$-$C_4$-haloalkoxy, phenyl, halophenyl or halogen,
and its N-oxides and addition salts with inorganic mineral acids, carboxylic acids or mononuclear arylsulfonic acids.

2. A process for the preparation of a hetarylalkene as claimed in claim 1, wherein a hetarylalkenal of the formula II

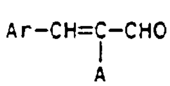 II

is reacted with an organolithium compound Li-B or with a Grignard reagent BMgHal, where Hal is halogen, to give a hetarylalkenol of the formula Ia

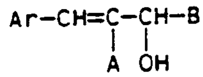

Ia

or a hetarylaldehyde Ar-CHO is reacted with a ketone

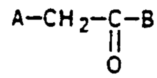

to give a hetarylalkenone of the formula Ib

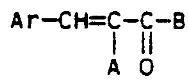

Ib

and the hetarylalkenone of the formula Ib is reduced using a method known per se to give a hetarylalkenol of the formula Ia.

3. A hetarylalkenal of the formula III

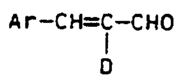

III

where Ar is 3-pyridyl and
D is $C_2$-$C_6$-alkyl, $C_3$-$C_8$-cycloalkyl, $C_3$-$C_8$-cycloalkyl-$C_1$-$C_3$-alkyl or mononuclear or dinuclear aryl-$C_1$-$C_3$-alkyl, in which aryl may be monosubstituted, disubstituted or trisubstituted by $C_1$-$C_4$-alkyl, $C_1$-$C_4$-alkoxy, $C_1$-$C_4$-haloalkyl, $C_1$-$C_4$-haloalkoxy, phenyl or halogen.

4. A process for the preparation of a hetarylalkenal of the formula III as claimed in claim 3, wherein two aldehydes, D-$CH_2$CHO and Ar-CHO, are reacted in a manner known per se to give a hetarylalkenal of the formula III.

5. A fungicide containing a hetarylalkene, an N-oxide or an acid addition salt as claimed in claim 1 and conventional additives.

6. A fungicide as claimed in claim 5, containing other active ingredients.

7. A method for controlling harmful fungi, wherein the fungi or the plants, seeds, materials or areas threatened by fungal attack are treated with a hetarylalkene I as claimed in claim 1 or with a fungicide as claimed in claim 5 or 6.

**Claims for the following Contracting States : GR, ES**

1. A process for the preparation of a hetarylalkene of the formula I

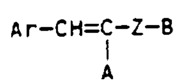

I

where
Ar is 3-pyridyl;
A is $C_1$-$C_6$-alkyl, $C_2$-$C_6$-alkenyl, $C_3$-$C_8$-cycloalkyl, $C_3$-$C_8$-cycloalkyl-$C_1$-$C_3$-alkyl or mononuclear, dinuclear or trinuclear aryl or aryl-$C_1$-$C_3$-alkyl, where aryl in each case may be monosubstituted, disubstituted or trisubstituted by $C_1$-$C_4$-alkyl, $C_1$-$C_4$-alkoxy, $C_1$-$C_4$-mono-, di- or trihaloalkyl, $C_1$-$C_4$-haloalkoxy, phenyl or

halogen;
Z is

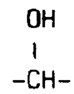,

B is mononuclear or dinuclear aryl or benzyl in which each of the aromatic rings may be monosubstituted, disubstituted or trisubstituted by $C_1$-$C_4$-alkyl, $C_1$-$C_4$-alkoxy, $C_1$-$C_4$-haloalkyl, $C_1$-$C_4$-haloalkoxy, phenyl, halophenyl or halogen,
and its N-oxides and addition salts with inorganic mineral acids, carboxylic acids or mononuclear arylsulfonic acids, wherein a hetarylalkenal of the formula II

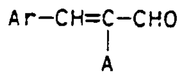

II

is reacted with an organolithium compound Li-B or with a Grignard reagent BMgHal, where Hal is halogen, to give a hetarylalkenol of the formula Ia

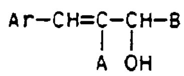

Ia

or a hetarylaldehyde Ar-CHO is reacted with a ketone

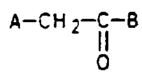

to give a hetarylalkenone of the formula Ib

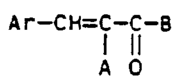

Ib

and the hetarylalkenone of the formula Ib is reduced using a method known per se to give a hetarylalkenol of the formula Ia.

2. A process for the preparation of a hetarylalkenal of the formula III

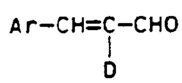

III

where Ar is 3-pyridyl and
D is $C_2$-$C_6$-alkyl, $C_3$-$C_8$-cycloalkyl, $C_3$-$C_8$-cycloalkyl-$C_1$-$C_3$-alkyl or mononuclear or dinuclear aryl-$C_1$-$C_3$-alkyl, in which aryl may be monosubstituted, disubstituted or trisubstituted by $C_1$-$C_4$-alkyl, $C_1$-$C_4$-alkoxy, $C_1$-$C_4$-haloalkyl, $C_1$-$C_4$-haloalkoxy, phenyl or halogen, wherein two aldehydes, D-$CH_2$CHO and Ar-CHO, are reacted in a manner known per se to give a hetarylalkenal of the formula III.

3. A fungicide containing a hetaryalkene, an N-oxide or an acid addition salt as claimed in claim 1 and conventional additives.

4. A fungicide as claimed in claim 3, containing other active ingredients.

5. A method for controlling harmful fungi, wherein the fungi or the plants, seeds, materials or areas threatened by fungal attack are treated with a hetarylalkene I as claimed in claim 1 or with a fungicide as claimed in claim 3 or 4.

## Revendications

**Revendications pour les Etats contractants suivants : DE, GB, FR, IT, NL, SE, LI, CH, BE, AT, DK**

**1.** Hétéroarylalcènes de formule générale I

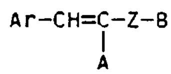

I

dans laquelle les symboles ont les significations suivantes :
Ar représente un groupe 3-pyridyle ;
A représente un groupe alkyle en C1-C6, alcényle en C2-C6, cycloalkyle en C3-C8, (cycloalyle en C3-C8)-alkyle en C1-C3, aryle ou aryl-alkyle en C1-C3 mono-, bi- ou tri-cyclique, dans lesquels le groupe aryle, dans les deux cas, peut porter un, deux ou trois substituants alkyle en C2-C4, alcoxy en C1-C4, mono-, di- ou tri-halogéno-alkyle en C1-C4, halognéoalcoxy en C1-C4, phényle ou halogéno;
Z représente

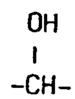

,

B représente un groupe aryle mono- ou bi-cyclique ou un groupe benzyle dans lesquels les noyaux aromatiques peuvent porter un, deux ou trois substituants alkyle en C1-C4, alcoxy en C1-C4, halogénoalkyle en C1-C4, halogénoalcoxy en C1-C4, phényle, halogéno-phényle ou halogéno;
ainsi que leurs N-oxydes et leurs sels formés par addtion avec des acides minéraux, des acides carboxyliques ou des acides arylsulfoniques monocycliques.

**2.** Procédé de préparation des hétéroarylalcènes de la revendication 1, caractérisé en ce que l'on fait réagri un hétéroarylalcénal de formule générale II

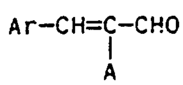

II

avec un dérivé organique du lithium Li-B ou un réactif de Grignard BMgHal dans lequel Hal représente un halogène, ce qui donne un hétéroarylalcénole formule générale Ia)

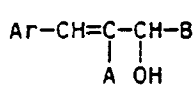

Ia

ou bien que l'on fait réagir un hétéroarylaldéhyde Ar-CHO avec une cétone

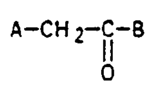

ce qui donne une hétéroarylalcénone de formule générale Ib)

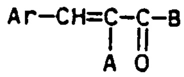 Ib

après quoi on réduit l'hétéroarylalcénone de formule générale Ib), par des procédés connus en soi en un hétéroarylalcénol de formule générale Ia).

**3.** Hétéroarylalcénals de formule générale III

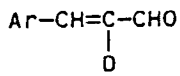 III

dans laquelle Ar représente un groupe 3-pyridyle et
D représente un groupe alkyle en C2-C6, cycloalkyle en C3-C8, (cycloalkyle en C3-C8)-alkyle en C1-C3 ou un groupe aryl-alkyle en C1-C3 mono- ou bicyclique, le groupe aryle pouvant porter un, deux ou trois substituants alkyle en C1-C4, alcoxy en C1-C4, halogéno-alkyle en C1-C4,halogénoalcoxy en C1-C4, phényle ou halogéno.

**4.** Procédé de préparation d'un hétéroarylalcénal de formule générale III de la revendication 3, caractérisé en ce que l'on fait réagir de manière connue en soi deux aldéhydes, D-$CH_2$CHO et Ar-CHO, ce qui donne un hétéroarylalcénal de formule générale III.

**5.** Produits fongicides contenant un hétéroarylalcène, un N-oxyde ou un sel d'acide selon la revendication 1, et des additifs usuels.

**6.** Produits fongicides selon la revendication 5, contenant d'autres constituants actifs.

**7.** Procédé pour combattre les mycètes nuisibles, caractérisé en ce que l'on traite les mycètes ou les végétaux, semences, matières ou surfaces menacés d'une attaque par les mycètes, par un hétéroarylalcène I de la revendication 1 ou par un produit fongicide selon la revendication 5 ou 6.

**Revendications pour les Etats contractants suivants : GR, ES**

**1.** Procédé de préparation des hétéroarylalcènes de formule générale I

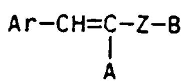 I

dans laquelle les symboles ont les significations suivantes :
Ar représente un groupe 3-pyridyle,
A représente un groupe alkyle en C1-C6, alcényle en C2-C6, cycloalkyle en C3-C8, (cycloalkyle en C3-C8)-alkyle en C1-C3, un groupe aryle ou aryl-alkyle en C1-C3 mono-, bi- ou tri-cyclique dans lesquels le groupe aryle, dans les deux cas, peut porter un, deux ou trois subsituants alkyles en C1-C4, alcoxy en C1-C4, mono-, di- ou tri-halogéno-alkyle en C1-C4, halogénoalcoxy en C1-C4, phényle ou halogéno;
Z représente

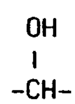 ,

B représente un groupe aryle mono- ou bi-cyclique ou un groupe benzyle dans lesquels les noyaux aromatiques peuvent porter un, deux ou trois substituants alkyles en C1-C4, alcoxy en C1-C4, halogénoalkyle en C1-C4, halogénoalcoxy en C1-C4, phényle, halogéno-phényle ou halogéno ;
et de leurs N-oxydes et sels formés par addition avec des acides minéraux, des acides carboxyliques ou des acides arylsulfoniques mono-cycliques, caractérisé en ce que l'on fait réagir un hétéroarylalcénal de

formule générale II

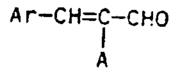

II

avec un dérivé organique de lithium Li-B ou un réactif de Grignard BMgHal dans lequel Hal représente un halogène, ce qui donne un hétéroarylalcénol de formule générale Ia)

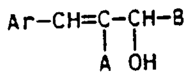

Ia

ou bien on fait réagir un hétéroarylaldéhyde Ar-CHO avec une cétone

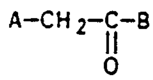

ce qui donne une hétéroarylalcénone de formule générale Ib

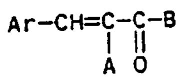

Ib

après quoi on réduit l'hétéroarylalcénone de formule générale Ib), par des procédés connus en soi, en l'hétéroarylalcénol de formule générale Ia).

2. Procédé de préparation d'un hétéroarylalcénal de formule générale III

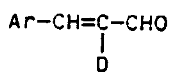

III

dans laquelle Ar représente un groupe 3-pyridyle et
D représente un groupe alkyle en C2-C6, cycloalkyle en C3-C8, (cycloalkyle en C3-C8)-alkyle en C1-C3 ou un groupe aryl-alkyle en C1-C3 mono- ou bicyclique dans lequel le groupa aryle peut porter un, deux ou trois subsitutuants alkyle en C1-C4, alcoxy en C1-C4, halogénoalkyle en C1-C4, halogénoalcoxy en C1-C4, phényle ou halogéno, caractérisé en ce que l'on fait réagir deux aldéhydes, $D-CH_2CHO$ et Ar-CHO, de manière connue en soi, ce qui donne l'hétéroarylalcénal de formule générale III.

3. Produits fongicides contenant un hétéroarylalcène, un N-oxyde ou un sel d'acide selon la revendication 1, et des additifs usuels.

4. Produits fongicides selon la revendication 3, contenant d'autres constituants actifs.

5. Procédé pour combattre les mycètes nuisibles, caractérisé en ce que l'on traite les mycètes ou les végétaux, semences, matières ou surfaces menacés d'une attaque par les mycètes, par un hétéroarylalcène I de la revendication 1 ou par un produit fongicide selon la revendication 3 ou 4.